# EUROPEAN PATENT APPLICATION

(11) **EP 1 160 331 A1**
(43) Date of publication of application: **05.12.2001**
(21) Application number: 01304646.1
(22) Date of filing: 25.05.2001
(51) Int. Cl.: C12P 17/10, C12P 17/16, C07D 231/52, C07D 239/46, C07D 239/48, C07D 403/12, C07D 405/04, C07D 405/14, C07D 413/14

(54) **Enzymatic oxidations**

(30) Priority: 31.05.2000 GB 0013368
(71) Applicant: Pfizer Limited, Sandwich, Kent CT13 9NJ (GB); PFIZER INC., New York, N.Y. 10017 (US)
(72) Inventor: Pacey, Michael Stephen, Sandwich, Kent CT13 9NJ (GB)
(74) Representative: Motion, Keith Robert

(57) **Abstract**

The following invention relates to a process for oxidising alkyl groups attached directly or via a linker, to a sulfonamide moiety (II) by the use of cytochrome P450 enzymes, to give the corresponding alcohol or carboxylic acid (I). 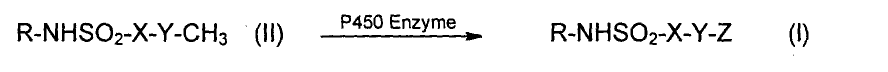 wherein R is an organic radical, X is a linker, Y is -C(CH₃)₂- or -CH(CH₃)- and Z is -CH₂OH or -COOH.

## Description

The following invention relates to a process for oxidising alkyl groups attached directly or via a linker, to a sulfonamide moiety by the use of a cytochrome P450 enzyme.

There are a large number of structurally complex molecules of interest to medicinal chemists, which possess a hydroxylated or carboxylated alkyl group, attached directly or via a linker, to a sulfonamide group. The synthesis of these molecules is complicated by the synthetic steps necessary to hydroxylate or carboxylate such alkyl groups whilst avoiding undesirable side reactions.

This problem has been solved by the process of the present invention, which teaches the use of a cytochrome P450 enzyme to selectively oxidise an alkyl group attached directly or via a linker to a sulfonamide group. The process which is specific and tolerant of other functional groups in the molecule allows the synthetic chemist to introduce a hydroxy or carboxy group into the synthesis of a molecule at a late stage, in a one step process. This reduces the overall number of steps required to synthesise medicinally important molecules and so improves the efficiency of their preparation.

The term 'Cytochrome P450' is used to describe a superfamily of hemoprotein enzymes. They are characterised by the absorption band of their FE"-CO complex form which has an absorption maxima at 447-452nm, indicative of a thiolate ligated hemoprotein. They are also called heme-thiolate proteins, though this category is much wider than just cytochrome P450. Cytochrome P450 appear to have diversified from a common ancestor and they may be found in almost all forms of living organism, from animals and plants to fungi and bacteria, where they carry out the role of an oxygenase.

Cytochrome P450 catalysed reactions are known to produce a range of metabolites. The reactions may usually be classified as one of three types:
a) Hydroxylation, the insertion of an oxygen atom between the H atom and some other heavier atom such as carbon or nitrogen,
b) Epoxidation, the addition of an oxygen atom to a carbon-carbon double bond,
c) Heteroatom oxidation, the addition of an oxygen atom to the electron pair on a heteroatom.

They are also known to catalyse reduction reactions. Useful reviews of the cytochrome P450 enzyme are provided in Handbook of Drug metabolism, Ed. Thomas Woolf, Publisher Marcel Decker, ISBN 0824702298, March 1999, chap 4, p109 and by T. Omura, Biochemical and Biophysical Research Communications, 1999, **266,** 690.

R. A. Johnson *et al.,* Biooganic Chemistry, 1973, **2,** 99, discloses the treatment of a number of dialkylbenzenes with the P450 enzyme containing microorganism *Sporotrichum sulfurescens,* to form an alcohol on the alkyl side chain.

The abstract of JP-60258173 discloses the synthesis of an α-(3-*t*-butyl-5-hydroxy-*t*-butylbenzylidene)butyrolactone by microbial hydroxylation of the corresponding *t*-butyl precursor. Suitable microorganisms for the transformation are those from the *Mucor* and *Aspergillus* strains.

C. Cerniglia *et al*., Applied and Environmental Microbiology, 1984, **47**, 111, discloses the transformation of 1- and 2-methylnaphthalene by *Cunninghamella elegans* into the corresponding hydroxymethyl derivative. A small amount of further metabolites were also isolated in which the product had been further oxidised to the carboxylic acid or where hydroxylation of the ring system had taken place.

H.L. Holland *et al.,* Tetrahedron Asymm., 1994, **5,** 1241, teaches the oxidation of chiral *para* substituted alkyl benzyl sulfides to the sulfoxide. In cases where the *para* substituent was *i*-propyl or *t*-butyl, hydroxylation at the terminal methyl of the alkyl group was seen, as well as oxidation of the sulfide. In the case of the *i*-propyl group, the corresponding sulfone was also seen. These transformations were conducted using the microorganism *Helminthosporium* NRRL-4671.

H. Schwartz *et al*., Appl. Microbiol. Biotechnol., 1994, **44,** 731, investigates the microbial oxidation of ebastine to carebastine. Of 15 microorganisms examined, only the *Cunninghamella* strains provided the desired biotransformation.

WO-A-99/47693 discloses a method for making fexofenadine from terfenadine by a biotransformation performed using a microorganism culture of the genus *Streptomyces* at a pH ranging between 5 and 8.

The oxidation by a cytochrome P450 enzyme of alkyl groups attached directly, or via a linker, to a sulfonamide moiety, is new. Sulfonamides are known to exhibit antimicrobial activity. Accordingly, molecules containing a sulfonamide group have not been viewed as suitable candidates for use in a biotransformation process as it was thought they would kill any microorganism used. Surprisingly, it has now been found that molecules containing a sulfonamide group may be used in certain oxidative biotransformations.

According to a first aspect of the present invention there is provided a process for synthesising the compounds of formula (I)

R-NHSO₂-X-Y-Z (I)

wherein
R is an organic radical;
X is selected from
   a) a 5- or 6- membered monocyclic aromatic ring optionally containing one or two heteroatoms, each independently selected from O, N and S;
   b) a C₁-C₆ alkylene group, straight chain or branched chain; and
   c) a direct link;
Y is -C(CH₃)₂- or -CH(CH₃)-; and
Z is -CH₂OH or -COOH;
which comprises oxidising a compound of formula (II)

R-NHSO₂-X-Y-CH₃ (II)

wherein R, X and Y are as defined above,
with a cytochrome P450 enzyme.

The process described may be used to oxidise a suitable alkyl group to the corresponding hydroxy or carboxy derivative. These oxidations proceed from the alkyl to the hydroxy and in the latter case, onwards from the hydroxy to the corresponding carboxylic acid. It will be appreciated that by careful manipulation of the reaction conditions, one may selectively isolate a product at the desired oxidation level and maximise the yield of the desired product. The oxidation may be stopped at the hydroxy derivative or allowed to continue onwards to the carboxylic acid. To produce the corresponding carboxylic acid no modification to the process is required other than that the process be given sufficient time to further oxidise the substrate. It should be noted that the nature of the R substituent may influence the speed of the reaction and hence the yields of the desired products. Whilst all P450 enzymes are considered suitable for conducting the disclosed invention, certain microorganisms will be particularly suitable for specific levels of oxidation.

This transformation is effected by a cytochrome P450 enzyme. Particularly favoured are microorganisms which contain the P450 enzyme. Preferred microorganisms are unicellular bacteria, exemplified by species such as *Escherichia coli,* filamentous bacteria exemplified by such strains as *Actinomyces* and *Streptomyces* and filamentous fungi. Suitable microorganisms are specifically noted in this text by their name and the American Type Culture Collection (ATCC) number assigned to them when deposited with a recognised International Depository Authority under the terms of the Budapest Treaty. Three new microorganisms were identified as being effective in this transformation and deposits were made with the American Type Culture Collection in Manassas, USA, under the terms of the Budapest Treaty. The Depositor was Pfizer Inc. (of 235 East 42nd Street, New York, New York, USA). Pfizer Ltd. is a wholly-owned subsidiary of Pfizer Inc.

All three are gram positive filamentous bacteria, belonging to the Actinomycetales: *Streptomyces species* PTA-1685, *Streptomyces cyaneus* PTA-1686 and *Streptomyces lydicus* PTA-1687 and were cultivated on quarter strength ATCC172 agar slope.

In a preferred embodiment X is selected from:
a) a 5- or 6- membered monocyclic aromatic ring optionally containing one or two heteroatoms, each independently selected from O, N and S; and
b) a C₁-C₆ alkylene group, straight chain or branched chain;

When X is as defined in (a) above, X may suitably be phenylene or a 5 or 6 membered aromatic heterocycle containing 1 heteroatom selected from O, N and S. Particularly suitably X may be phenylene or a 6 membered aromatic heterocycle containing 1 heteroatom selected from O, N and S. Most suitably X is phenylene or pyridylene.

When X is as defined in (b) above, X may suitably be a C₂₋₄ alkylene group, straight chain or branched chain. Most suitably X is ethylene or propylene.

It is believed that this process is specific to the oxidation of alkyl groups, attached directly or via a linker to a sulfonamide moiety, and that this reaction will be tolerated by a wide range of R groups. Accordingly R may encompass all organic radicals. Preferably, R is a 5- or 6- membered monocyclic aromatic ring optionally containing one or two heteroatoms, each independently selected from O, N and S, said ring being optionally further substituted. Particularly preferred is a 5- or 6- membered monocyclic aromatic ring containing two heteroatoms, each independently selected from O and N, said ring being optionally further substituted. Most preferred are substituted pyrazoles, substituted pyrimidines and substituted isoxazoles. The invention has been utilised with a variety of differing R substituents; suitable R substituents include:

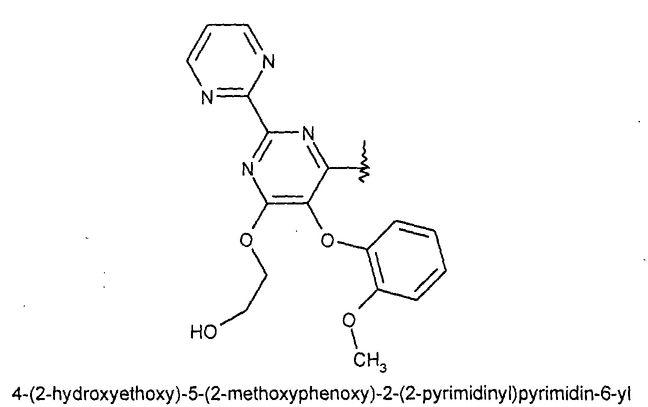

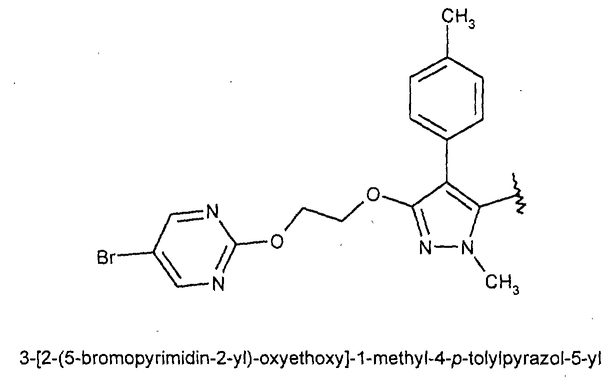

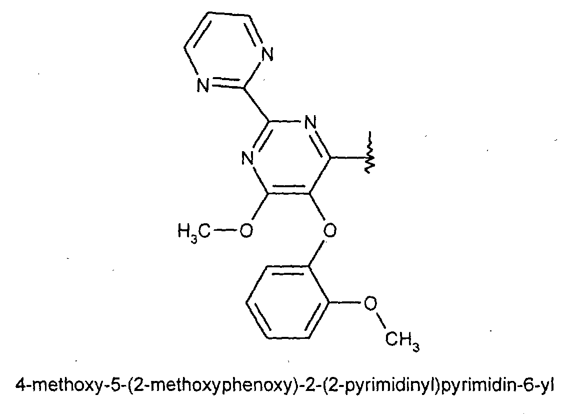

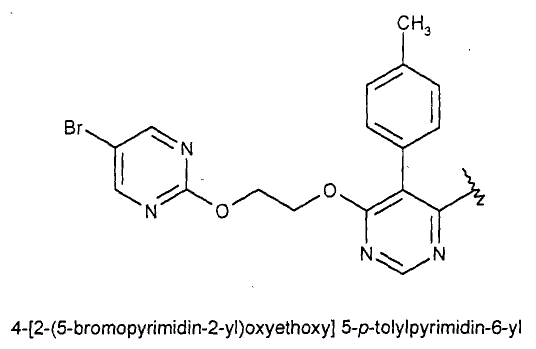

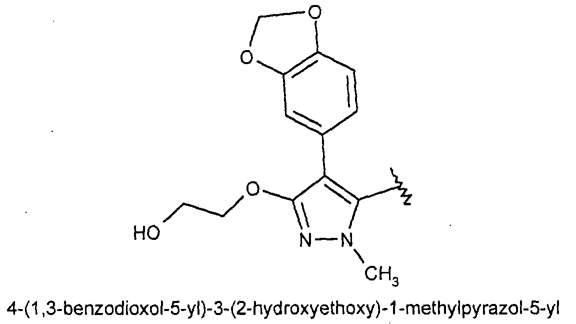

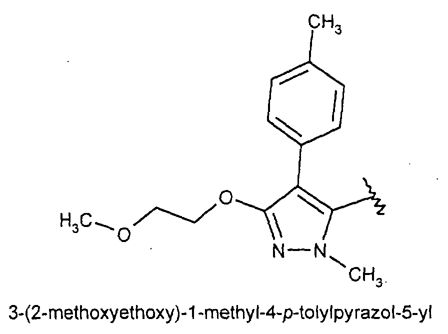

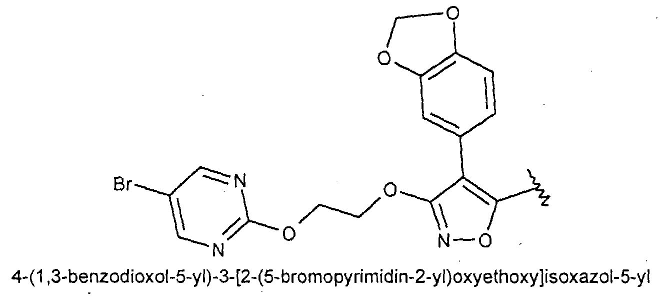

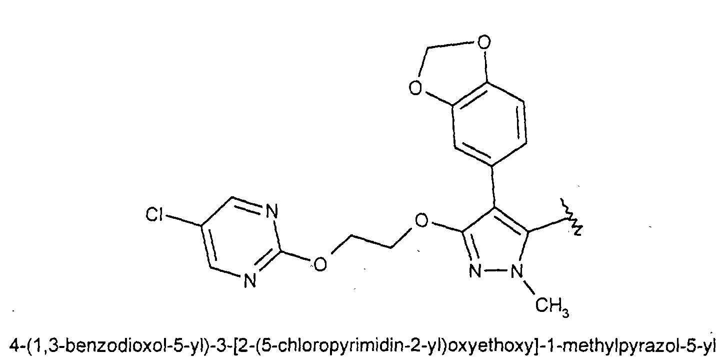

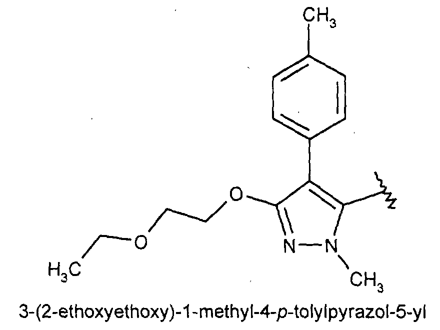

One embodiment of the present invention is a process for making compounds of formula (I) when Z is -CH₂OH, Y is -C(CH₃)₂- and X and R are as defined for formula (I).

According to this embodiment, preferably X is phenylene, pyridylene, ethylene or propylene; most preferably X is 1,4-phenylene.

According to this embodiment R is preferably selected from one of:
3-[2-(5-bromopyrimidin-2-yl)oxyethoxy]-1-methyl-4-*p*-tolylpyrazol-5-yl
4-[2-(5-bromopyrimidin-2-yl)oxyethoxy]-5-*p*-tolylpyrimidin-6-yl
4-(2-hydroxyethoxy)-5-(2-methoxyphenoxy)-2-(2-pyrimidinyl)pyrimidin-6-yl
4-methoxy-5-(2-methoxyphenoxy)-2-(2-pyrimidinyl)pyrimidin-6-yl
3-(2-ethoxyethoxy)-1-methyl-4-*p*-tolylpyrazol-5-yl
4-(1,3-benzodioxol-5-yl)-3-[2-(5-chloropyrimidin-2-yl)oxyethoxy]-1-methylpyrazol-5-yl
4-(1,3-benzodioxol-5-yl)-3-(2-hydroxyethoxy)-1-methylpyrazol-5-yl
4-(1,3-benzodioxol-5-yl)-3-[2-(5-bromopyrimidin-2-yl)oxyethoxy]isoxazol-5-yl
Most preferably R is 4-[2-(5-bromopyrimidin-2-yl)oxyethoxy]-5-*p*-tolylpyrimidin-6-yl

In this embodiment of the invention, micro-organisms particularly suitable for use in the process include: *Streptomyces lavendulae* ATCC14159, *Streptomyces* sp. PTA-1685, *Streptomyces cyaneus* PTA-1686, *Streptomyces lydicus* PTA-1687, *Streptomyces griseus* ATCC55070, *Streptomyces griseolus* ATCC11796, *Amyclatopsis orientalis* ATCC19795, *Streptomyces griseus* subsp. *griseus* ATCC13273, *Streptomyces argentolus* ATCC11009, *Nocardia meditteranei* ATCC21271, *Streptomyces fumanus* ATCC19904, *Amycolata autotrophica* ATCC35203, *Streptomyces rimosus* subsp. *rimosus* ATCC10970, *Streptomyces griseus* subsp. *griseus* ATCC10137, *Streptomyces* sp. ATCC31273, *Cunninghamella echinulata* var. *elegans* ATCC8688a, *Mortierella isabellina* ATCC42613, *Verticillium lecanii* ATCC60540, *Mucor circinelloides* ATCC7941, *Cunninghamella echinulata* var. *echinulata* ATCC36190, *Syncephalastrum racemosum* ATCC18192, *Beauvaria sulphurescens* ATCC7159, *Absidia pseudocylindrospora* ATCC24169, *Amycolata autotrophica* ATCC13181, *Rhodococcus rhodochrous* ATCC12674, *Rhodococcus rhodochrous* ATCC19067, *Bacillus megaterium* ATCC14581, *Bacillus megaterium* ATCC13368, *Rhodococcus* sp. ATCC19070, *Actinomyces* sp. ATCC53828, *Bacillus subtilis* ATCC55060, *Pseudomonas putida* ATCC17453, *Pseudomonas putida* ATCC49451, *Bacillus sphaericus* ATCC10208, *Rhizopus oryzae* ATCC11145, *Absidia blakesleeeana* ATCC10148a, *Sepedonium chrysospermum* ATCC13378, *Alcaligenes eutrophus* ATCC17697, *Streptomyces galilaeus* ATCC31133, *Actinoplanes missouriensis* ATCC23342, *Actinoplanes missouriensis* ATCC14538, *Streptomyces peucetius* subsp. *caesius* ATCC27952, *Streptomyces lincolnensis* ATCC25466, *Streptomyces bambergiensis* ATCC13879, *Streptomyces argillaceus* ATCC12956, *Streptomyces albogriseolus* ATCC31422, *Streptomyces rutgersensis* ATCC3350, *Corynebacterium hydrocarboxydans* ATCC21767, *Streptomyces fradiae* ATCC10745, *Streptomyces hydrogenans* ATCC19631, Unidentified bacterium ATCC13930, *Actinoplanes* sp. ATCC53771, *Thamnidium elegans* ATCC18191, *Aspergillus terreus* ATCC10020, *Curvularia lunata* ATCC13432, *Emericella unguis* ATCC13431, *Epicoccum humicola* ATCC12722, *Rhodococcus chlorophenolicus* ATCC49826, *Aspergillus ochraceus* ATCC18500, *Pithomyces cynodontis* ATCC26150, *Streptomyces roseochromogenes* ATCC13400, *Streptomyces griseus* subsp. *autotrophica* ATCC53668, *Streptomyces griseus* subsp. *griseus* ATCC23337, *Absidia repens* ATCC14849 and *Aspergillus alliaceus* ATCC10060.

Preferred microorganisms for this embodiment where R is 3-[2-(5-bromopyrimidin-2-yl)oxyethoxy]-1-methyl-4-*p*-tolylpyrazol-5-yl include: *Syncephalastrum racemosum* ATCC18192, *Streptomyces* sp. PTA-1685, *Streptomyces lavendulae* ATCC14159, *Streptomyces cyaneus* PTA-1686, *Streptomyces griseus* ATCC55070, *Amycolatopsi*s *orientalis* ATCC 19795, *Streptomyces argentolus* ATCC11009, *Nocardia meditteranei* ATCC21271, *Streptomyces fumanus* ATCC19904, *Streptomyces rimosus* subsp. *rimosus* ATCC10970, *Streptomyces griseus* ATCC10137, *Cunninghamella echinulata* ATCC8688a, *Mortierella isabellina* ATCC42613, *Verticillium lecanii* ATCC60540, *Mucor circinelloides* ATCC7941 and *Cunninghamella echinulata* ATCC10028b.

Preferred microorganisms for this embodiment where R is 4-[2-(5-bromopyrimidin-2-yl)oxyethoxy]-5-p-tolylpyrimidin-6-yl include: *Streptomyces lavendulae* ATCC14159, *Streptomyces* sp. PTA-1685, *Streptomyces cyaneus* PTA-1686, *Streptomyces lydicus* PTA-1687, *Streptomyces griseus* ATCC55070, *Streptomyces griseolus* ATCC11796, *Amyclatopsis orientalis* ATCC19795, *Streptomyces griseus* subsp. *griseus* ATCC13273, *Streptomyces fumanus* ATCC19904, *Amycolata autotrophica* ATCC35203, *Streptomyces griseus* subsp. *griseus* ATCC10137, *Streptomyces* sp. ATCC31273, *Mortierella isabellina* ATCC42613, *Verticillium lecanii* ATCC60540, *Mucor circinelloides* ATCC7941, *Cunninghamella echinulata* var. *echinulata* ATCC36190, *Syncephalastrum racemosum* ATCC18192, *Amycolata autotrophica* ATCC13181, *Rhodococcus rhodochrous* ATCC12674, *Rhodococcus rhodochrous* ATCC19067, *Bacillus megaterium* ATCC14581, *Bacillus megaterium* ATCC13368, *Rhodococcus* sp. ATCC19070, *Actinomyces* sp. ATCC53828, *Bacillus subtilis* ATCC55060, *Pseudomonas putida* ATCC49451, *Bacillus sphaericus* ATCC10208, *Rhizopus oryzae* ATCC11145, *Absidia blakesleeeana* ATCC10148a, *Sepedonium chrysospermum* ATCC13378, *Alcaligenes eutrophus* ATCC17697, *Streptomyces galilaeus* ATCC31133, *Actinoplanes missouriensis* ATCC23342, *Actinoplanes missouriensis* ATCC14538, *Streptomyces peucetius* subsp. *caesius* ATCC27952, *Streptomyces lincolnensis* ATCC25466, *Streptomyces bambergiensis* ATCC13879, *Streptomyces argillaceus* ATCC12956, *Streptomyces albogriseolus* ATCC31422, *Streptomyces rutgersensis* ATCC3350, *Corynebacterium hydrocarboxydans* ATCC21767, *Streptomyces fradiae* ATCC10745, *Streptomyces hydrogenans* ATCC19631, Unidentified bacterium ATCC13930, *Actinoplanes* sp. ATCC53771, *Thamnidium elegans* ATCC18191, *Aspergillus terreus* ATCC10020, *Curvularia lunata* ATCC13432, *Emericella unguis* ATCC13431 *Epicoccum humicola* ATCC12722, *Rhodococcus chlorophenolicus* ATCC49826, *Aspergillus ochraceus* ATCC18500, *Streptomyces roseochromogenes* ATCC13400, *Streptomyces griseus* subsp. *autotrophica* ATCC53668, *Streptomyces griseus* subsp. *griseus* ATCC23337, *Absidia repens* ATCC14849 and *Aspergillus alliaceus* ATCC10060.

Preferred microorganisms for this embodiment where R is 4-(2-hydroxyethoxy)-5-(2-methoxyphenoxy)-2-(2-pyrimidinyl)pyrimidin-6-yl include: *Streptomyces rimosus* subsp. *rimosus* ATCC10970, *Streptomyces fumanus* ATCC19904, *Streptomyces argentolus* ATCC11009, *Bacillus megaterium* ATCC14538, *Streptomyces griseus* ATCC13273, *Streptomyces griseus* ATCC10137, *Streptomyces griseolus* ATCC11796, *Streptomyces lavendulae* ATCC14159, *Streptomyces cyaneus* PTA-1686, *Streptomyces* sp. PTA-1685, *Amycolata autotrophica* ATCC13181, *Amycolata autotrophica* ATCC35203 and *Mortierella isabellina* ATCC42613.

Preferred micro-organisms for this embodiment where R is 4-(1,3-benzodioxol-5-yl)-3-[2-(5-bromopyrimidin-2-yl)oxyethoxy]isoxazol-5-yl include: *Streptomyces rimosus* subsp. *rimosus* ATCC10970, *Streptomyces fumanus* ATCC19904, *Streptomyces argentolus* ATCC11009, *Bacillus megaterium* ATCC14538, *Streptomyces griseus* ATCC13273, *Streptomyces griseus* ATCC10137, *Streptomyces griseolus* ATCC11796, *Streptomyces lavendulae* ATCC14159, *Streptomyces cyaneus* PTA-1686, *Streptomyces* sp. PTA-1685, *Amycolata autotrophica* ATCC13181, *Amycolata autotrophica* ATCC35203 and *Mortierella isabellina* ATCC42613.

Another embodiment of this aspect of the present invention is a process for making compounds of formula I when Z is -CH₂OH, Y is -CH(CH₃)- and X and R are as defined in formula (I).

According to this embodiment, preferably X is phenylene, pyridylene, ethylene or propylene; most preferably X is 2,5-pyridylene, wherein Y is at the 2-position.

According to this embodiment, R is preferably 4-(1,3-benzodioxol-5-yl)-3-[2-(5-chloropyrimidin-2-yl)oxyethoxy]-1 -methylpyrazol-5-yl.

Micro-organisms particularly suitable for use in this embodiment include: *Streptomyces lavendulae* ATCC14159, *Streptomyces* sp. PTA-1685, *Streptomyces cyaneus* PTA-1686, *Streptomyces lydicus* PTA-1687, *Streptomyces griseus* ATCC55070, *Streptomyces griseolus* ATCC11796, *Amyclatopsis orientalis* ATCC 19795, *Streptomyces griseus* subsp. *griseus* ATCC 13273, *Streptomyces argentolus* ATCC11009, *Nocardia meditteranei* ATCC21271, *Streptomyces fumanus* ATCC19904, *Amycolata autotrophica* ATCC35203, *Streptomyces rimosus* subsp. *rimosus* ATCC10970, *Streptomyces griseus* subsp. *griseus* ATCC10137, *Streptomyces* sp. ATCC31273, *Cunninghamella echinulata* var. *elegans* ATCC8688a, *Mortierella isabellina* ATCC42613, *Verticillium lecanii* ATCC60540, *Mucor circinelloides* ATCC7941, *Cunninghamella echinulata* var. *echinulata* ATCC36190, *Syncephalastrum racemosum* ATCC18192, *Beauvaria sulphurescens* ATCC7159, *Absidia pseudocylindrospora* ATCC24169, *Amycolata autotrophica* ATCC13181, *Rhodococcus rhodochrous* ATCC12674, *Rhodococcus rhodochrous* ATCC19067, *Bacillus megaterium* ATCC14581, *Bacillus megaterium* ATCC13368, *Rhodococcus* sp. ATCC19070, *Actinomyces* sp. ATCC53828, *Bacillus subtilis* ATCC55060, *Pseudomonas putida* ATCC17453, *Pseudomonas putida* ATCC49451, *Bacillus sphaericus* ATCC10208, *Rhizopus oryzae* ATCC11145, *Absidia blakesleeeana* ATCC10148a, *Sepedonium chrysospermum* ATCC13378, *Alcaligenes eutrophus* ATCC17697, *Streptomyces galilaeus* ATCC31133, *Actinoplanes missouriensis* ATCC23342, *Actinoplanes missouriensis* ATCC14538, *Streptomyces peucetius* subsp. *caesius* ATCC27952, *Streptomyces lincolnensis* ATCC25466, *Streptomyces bambergiensis* ATCC13879, *Streptomyces argillaceus* ATCC12956, *Streptomyces albogriseolus* ATCC31422, *Streptomyces rutgersensis* ATCC3350, *Cotynebacterium hydrocarboxydans* ATCC21767, *Streptomyces fradiae* ATCC10745, *Streptomyces hydrogenans* ATCC19631, Unidentified bacterium ATCC13930, *Actinoplanes* sp. ATCC53771, *Thamnidium elegans* ATCC18191, *Aspergillus terreus* ATCC10020, *Curvularia lunata* ATCC13432, *Emericella unguis* ATCC13431, *Epicoccum humicola* ATCC12722, *Rhodococcus chlorophenolicus* ATCC49826, *Aspergillus ochraceus* ATCC18500, *Pithomyces cynodontis* ATCC26150, *Streptomyces roseochromogenes* ATCC13400, *Streptomyces griseus* subsp. *autotrophica* ATCC53668, *Streptomyces griseus* subsp. *griseus* ATCC23337, *Absidia repens* ATCC14849 and *Aspergillus alliaceus* ATCC10060.

The oxidation of the alkyl group to the corresponding hydroxy derivative using a P450 enzyme may be allowed to continue onwards to the carboxylic acid. No modification to the process is required other than that the process be given sufficient time to further oxidise the substrate. It will be appreciated that by careful manipulation of the reaction conditions, one may selectively isolate a product at the desired oxidation level and maximise the yield of the desired product. Further, the nature of the R substituent may influence the speed of the reaction and hence the yields of the desired products. Whilst all P450 enzymes are considered suitable for conducting the disclosed invention, certain microorganisms will be particularly suitable for specific levels of oxidations.

Another embodiment of the present invention is a process for making compounds of formula (I) when Z is -COOH, Y is -C(CH₃)₂- and X and R are as defined in formula (I).

In this embodiment, preferably X is phenylene, pyridylene, ethylene or propylene; most preferably X is 1,4-phenylene.

According to this embodiment, R is preferably selected from one of;
3-[2-(5-bromopyrimidin-2-yl)oxyethoxy]-1-methyl-4-*p*-tolylpyrazol-5-yl
4-[2-(5-bromopyrimidin-2-yl)oxyethoxy]-5-*p*-tolylpyrimidin-6-yl
1-methyl-3-(2-methoxyethoxy)-4-*p*-tolylpyrazol-5-yl
3-(2-ethoxyethoxy)-1-methyl-4-*p*-tolylpyrazol-5-yl.
Most preferably R is 4-[2-(5-bromopyrimidin-2-yl)oxyethoxy]-5-*p*-tolylpyrimidin-6-yl.

Microorganisms particularly suitable for use in this embodiment include: *Amycolata autotrophica* ATCC 35203, *Nocardia meditteranei* ATCC21271, *Amycolatopsis orientalis* ATCC19795 *Streptomyces griseolus* ATCC11796, *Streptomyces rimosus* subsp. *rimosus* ATCC10970 and *Nocardia meditteranei* ATCC21271.

Preferred microorganisms for this embodiment where R is 3-[2-(5-bromopyrimidin-2-yl)oxyethoxy]-1-methyl-4-*p*-tolylpyrazol-5-yl include: *Amycolata autotrophica* ATCC 35203, *Nocardia meditteranei* ATCC21271 and *Amycolatopsis orientalis* ATCC19795

Preferred micro-organisms for this embodiment where R is 4-[2-(5-bromopyrimidin-2-yl)oxyethoxy]-5-*p*-tolylpyrimidin-6-yl include: *Streptomyces griseolus* ATCC11796, *Streptomyces rimosus* subsp. *rimosus* ATCC10970 and *Nocardia meditteranei* ATCC21271.

A further embodiment of the present invention is a process for making compounds of formula (I) when Z is -COOH, Y is -CH(CH₃)- and X and R are as defined in formula (I).

According to this embodiment, preferably X is phenylene, pyridylene, ethylene or propylene; most preferably X is 2,5-pyridylene.

Microorganisms particularly suitable for use in this embodiment include *Amycolata autotrophica* ATCC 35203, *Nocardia meditteranei* ATCC21271, *Amycolatopsis orientalis* ATCC19795 *Streptomyces griseolus* ATCC11796, *Streptomyces rimosus* subsp. *rimosus* ATCC10970 and *Nocardia meditteranei* ATCC21271.

The introduction of a functional group into a complex molecule that already possesses a wide range of functionality is extremely difficult. Unless one has absolute specificity it is likely that the reaction will be accompanied by a host of competing side reactions. The effect of this is to produce the desired compound in a smaller yield and to provide difficulties in extracting and purifying the desired molecule. These problems are particularly acute in the case of oxidising a C-H bond. This is almost impossible to achieve selectively using standard chemical means.

To avoid this problem, the skilled synthetic chemist will employ a careful retrosynthetic analysis of the synthetic target. The synthetic strategy will involve avoiding side reactions and will usually involve a protecting group strategy. In avoiding conflicting side reactions, the chemist will be forced to add extra reaction steps to his synthesis, e.g. protection and deprotection.

These extra steps are undesirable. There are the additional costs of the reagents, solvents and other manufacturing costs. The extra steps will also depress the overall yield, again adding to the cost of the final product. Further there are the problems associated with purifying the compound after each step and disposing of any pollutants.

The process of the present invention solves these problems by introducing a hydroxy or carboxy moiety into a molecule with great specificity in one step with high yield. So specific is the process, that such moieties may be introduced at a very late stage, into a molecule with a multiplicity of other functionality. The advantage of this process are that fewer synthetic steps are required leading to lower costs in reagents, solvents and production costs. Further there will be fewer pollutants resulting in less environmental damage and clean up costs. The process is also suitable for production on an industrial scale.

Microorganisms containing these P450 cytochrome enzymes suitable for use in this invention may be obtained from a variety of sources. They may be isolated from various soil samples, then grown on various agars as described in the ATCC Catalogue of Bacteria and Bacteriophages, 18^{th} Edition, 1992, or the ATCC Catalogue of Fungi and Yeasts, 17^{th} Edition, 1987.

The processes of the invention may be carried out in any suitable aqueous fermentation medium.

It will be appreciated that the process of the present invention may be conducted in the presence of various additives such as solubilisers such as alcohols and dipolar aprotic solvents, e.g. methanol and dimethylsulfoxide.

The wide range of functionality able to tolerate the process of the present invention is exemplified in the following examples;

High performance liquid chromatography (HPLC) retention times and UV spectra were recorded using a Hewlett-Packard 1090 LUSI diode-array spectrophotometer (method A). All NMR spectra were measured in CDCl₃ or MeOD by an Inova 300 MHz or 400 MHz spectrometer unless otherwise indicated and peak positions are expressed in parts per million (ppm) downfield from tetramethylsilane. The peak shapes are denoted as follows: s, singlet; d, doublet; t, triplet; q, quartet; m, multiplet; br broad. High resolution MS data was acquired on a AutoSpecQ with electrospray ionisation (ESI) or thermospray ionisation (TSPI) using a PEG reference (or on a Bruker Apex II FTMS with ESI where indicated).

Infra red data was collected on a Perkin Elmer Paragon 1000 FT-IR using conventional techniques such as a polyethylene (PE) film or as a NUJOL dispersion on sodium chloride (NaCI) discs. Peak positions are expressed in cm⁻¹.

HPLC-MS data was acquired using a Hewlett-Packard 1090M liquid chromatograph interfaced to a VG platform II mass spectrometer equipped with an ES source (method B).

| HPLC method A: | |
|---|---|
| Column | Beckman Ultrasphere™ 5 micron ODS 4 mm x 25cm |
| Mobile Phase | Linear gradient: methanol:water (65:35) to methanol:water (95:5) over 40 minutes |
| Flow rate | 0.85ml/min |

| HPLC-MS method B: | |
|---|---|
| Column | Phenomenex Magellan™ 5 micron ODS 4.6mm x 15cm |
| Mobile Phase | Gradient: 0.1% v/v trifluoroacetic acid (TFA) in water:acetonitrile (90:10) to 0.1%TFA in water:acetonitrile (2:98) over 5 minutes, maintain 0.1%TFA in water:acetonitrile (2:98) 5 to 11.5 minutes. |
| Flow rate | 0.85ml/min |

Use is made of the following fermentation media.

| AS-7H inoculum medium | |
|---|---|
| Cornstarch (Hidex™) | 20g |
| Cotton Seed Meal (Pharmamedia™) | 15g |
| Ardamine pH™ | 5g |
| Calcium carbonate | 2g |
| Tap water | 1l |
| NaOH | To pH 7.2 |

| AP-5H Production Medium | |
|---|---|
| Cornststarch | 80g |
| Yeast extract (Oxoid™) | 5g |
| K₂HPO₄ | 1g |
| MgSO₄.7H₂O | 1g |
| Glutamic acid | 1g |
| | |
| FeSO₄. 7H₂O | 0.01g |
| ZnSO₄.2H₂O | 0.001g |
| MnSO₄.H₂O | 0.001g |
| CaCO₃ | 7g |
| Tap water | 1l |
| NaOH | To pH 7.0 |

| MY Inoculum and Production Medium | |
|---|---|
| Glucose | 10g |
| Peptone (Difco™) | 5g |
| Yeast extract (Oxoid™) | 3g |
| Malt extract (Oxoid™) | 5g |
| | |
| Tap water | 1l |
| NaOH | To pH 6.3 - 6.5 |

| Tomato Inoculum and Production Medium | |
|---|---|
| Tomato Paste | 40g |
| Corn Steep Liquor | 2.5g |
| Oats | 10g |
| Cerelose | 10g |
| FeSO₄. 7H₂O | 0.001 g |
| ZnSO₄.2H₂O | 0.001g |
| MnSO₄.H₂O | 0.001g |
| Demineralised Water | 1l |
| NaOH | To pH 6.8 |

### Example 1 ― Preparation and Isolation of N-[3-{2-[(5-bromo-2-pyrimidinyl)oxy]ethoxy}-1-methyl-4-(4-methylphenyl)-1H-pyrazol-5-yl]-4-(2-hydroxy-1,1 -dimethylethyl)benzenesulfonamide

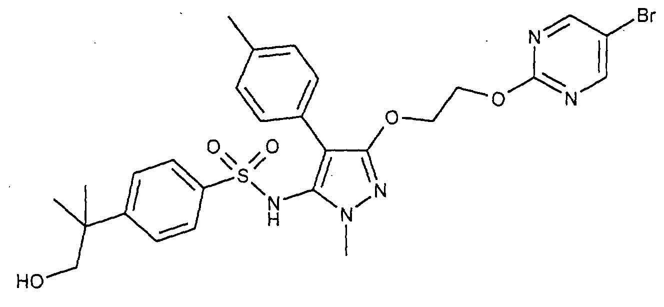

*Streptomyces rimosus* subsp. *rimosus* ATCC10970 maintained on a quarter strength ATCC172 agar slope was inoculated as a loopful of spores into a 300 ml Erlenmeyer flask containing 50ml of AS-7H inoculum medium. This was allowed to incubate for 2 days at 28°C, 200rpm on an Infors Multitron™ Shaker with 1" throw. 2mls of this inoculum medium was then transferred to each of sixteen 300ml Erlenmeyer flask containing 50ml of AP-5H production medium and incubated under the same conditions for a further 24 hours. At this point 20mg of N-[3-{2-[(5-bromo-2-pyrimidinyl)oxy]ethoxy}-1-methyl-4-(4-methylphenyl)-1*H*-pyrazol-5-yl]-4-(*tert*-butyl)benzenesulfonamide (GB Patent Application No 9917858.4) dissolved in 0.5ml of methanol was added to each of the flasks and the fermentation allowed to continue under the same conditions for a further 6 hours. Each flask was then extracted with 200mls of ethyl acetate and the combined ethyl acetate solubles concentrated to dryness to give a gum solid, 1.4 g.

The crude extract was purified by preparative reversed phase HPLC with a Phenomenex Magellen™ 5µ C18 column (150mm x 21.2mm) in three injections. Using a gradient mobile phase of 35:65 to 20:80 (0.1% trifluoroacetic acid in water):methanol from 1.5 to 20 minutes at a flow rate of 20ml/min, the product was eluted at 11.4min. The fractionation was repeated with another three injections using a gradient mobile phase of 35:65 to 20:80 water:methanol at a flow rate of 20ml/min. The compound of interest eluted again at 11.4min and the product fractions were concentrated under reduced pressure to yield the title compound as a colourless amorphous solid (53.5mg).
HPLC retention time ― Method A; 11.3 minutes.

δH (300MHz, CDCl₃)(selected nmr data) 8.45 (2H, s); 7.40 (2H, d); 7.15 (1H, s); 7.10 (2H, d); 6.80 (4H, s); 4.65 (2H, m); 4.55 (2H, m); 3.80 (3H, s); 3.50 (2H,s ); 2.20 (3H, s); 1.15 (6H, s)
m/z (ESI) [M+H]⁺ = 616.1216, C₂₇H₃₁BrN₅O₅S requires 616.1229
m/z (ESI) [M+Na]⁺ = 638.1060, C₂₇H₃₀BrN₅O₅SNa requires 638.1049

### Example 2 ― Preparation and Isolation of 2-[4-({[3-{2-[(5-bromo-2-pyrimidinyl)oxy]ethoxy}-1-methyl-4-(4-methylphenyl)-1H-pyrazol-5-yl]amino}sulfonyl)phenyl]-2-methylpropanoic acid

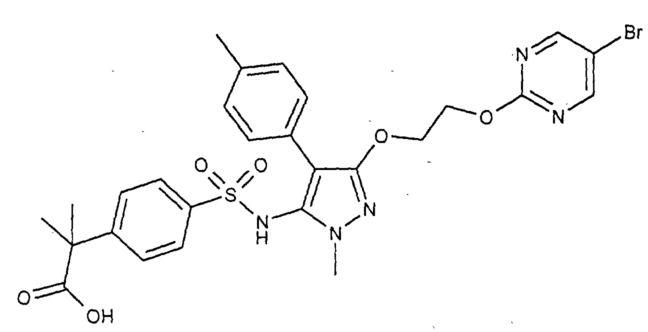

*Amycolata autotrophica* ATCC35203 maintained on a quarter strength ATCC172 agar slope was inoculated as a loopful of spores into five 300 ml Erlenmeyer flasks each containing 50ml of MY inoculum medium. This was allowed to incubate for 2 days at 28°C, 200rpm on an Infors Multitron™ Shaker with 1" throw. Two mls of this inoculum was then transferred to each of sixty 300ml Erlenmeyer flask containing 50ml of MY production medium and incubated under the same conditions for a further 24 hours. At this point 20mg of N-[3-{2-[(5-bromo-2-pyrimidinyl)oxy]ethoxy}-1-methyl-4-(4-methylphenyl)-1*H*-pyrazol-5-yl]-4-(*tert*-butyl)benzenesulfonamide (GB Patent Application No 9917858.4) dissolved in 0.5ml of methanol was added to each of the flasks and the fermentation allowed to continue under the same conditions for a further 6 hours. Each flask was then extracted with 200mls of ethyl acetate and the combined ethyl acetate solubles concentrated to dryness to give a gum solid, 2.0 g.

The crude extract was purified by preparative reversed phase HPLC with a Phenomenex Magellen™ 5µ C18 column (150mm x 21.2mm) in six injections. Using a gradient mobile phase of 35:65 to 20:80 water:methanol from 1.5 to 20 minutes at a flow rate of 20ml/min, the product was eluted at 12.5min. The product fractions were concentrated under reduced pressure to yield the title compound as a colourless amorphous solid (213.4 mg).
HPLC retention time ― Method A; 11.5 minutes.

δH (300MHz, CDCl₃)(selected nmr data) 8.65 (2H, s); 7.15 (4H, m); 6.95 (2H, d); 6.90 (s,1H); 6.70 (2H, d); 4.65 (2H, m); 4.45 (2H, m); 3.90 (3H, s); 2.30 (3H, s); 1.55 (6H, s)
m/z (ESI) [M+H]⁺ = 630.1005, C₂₇H₂₉BrN₅O₆S requires 630.1022
m/z (ESI) [M+Na]⁺ = 652.0842, C₂₇H₂₈BrN₅O₆SNa requires 652.0841
νₘₐₓ (NaCl, film) 3241, 2976, 1710, 1572, 1551, 1427, 1327, 1167, 1085

### Example 3 ― Preparation of N-[6-{2-[(5-bromo-2-pyrimidinyl)oxy]ethoxy}-5-(4-methylphenyl)-4-pyrimidinyl]-4-(2-hydroxy-1,1-dimethylethyl)benzenesulfonamide

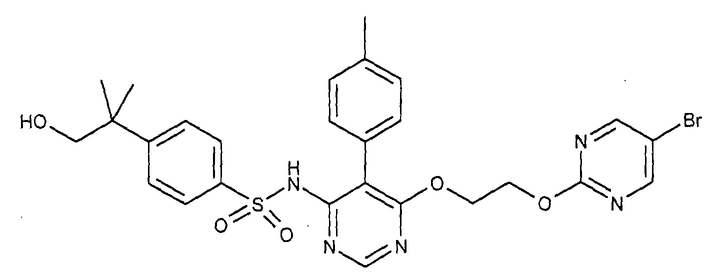

*Streptomyces rimosus* subsp. *rimosus* ATCC10970 maintained on a quarter strength ATCC172 agar slope was inoculated as a loopful of spores into a 300 ml Erlenmeyer flask containing 50ml of AS-7H inoculum medium. This was allowed to incubate for 2 days at 28°C, 200rpm on an Infors Multitran™ Shaker with 1" throw. 2mls of this inoculum medium was then transferred to each of six 300ml Erlenmeyer flask containing 50ml of AP-5H production medium and incubated under the same conditions for a further 24 hours. At this point 5mg of N-[6-{2-[(5-bromo-2-pyrimidinyl)oxy]ethoxy}-5-(4-methylphenyl)-4-pyrimidinyl]-4-(*tert*-butyl)benzenesulfonamide (EP 0658548B1) dissolved in 0.5ml of dimethylsulfoxide was added to each of the flasks and the fermentation allowed to continue under the same conditions for a further 3 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm). Using a gradient mobile phase of 60:40 to 5:95 water:acetonitrile from 2 to 14 minutes at a flow rate of 20ml/min, the product was eluted at 8.0min. The product fractions were concentrated under reduced pressure to yield the title compound as a colourless amorphous solid (1.45mg).
HPLC retention time ― Method A; 12.5 minutes.

δH (300MHz, CDCl₃)(selected nmr data) 8.45 (2H, s); 8.40 (1H, s); 8.05 (2H, d); 7.55 (2H, d); 7.20 (2H, d); 7.10 (2H, d); 4.65 (2H, m); 4.60, (2H, m); 3.65 (2H, s); 2.40 (3H, s); 1.35 (6H, s)
m/z (ESI, FTMS) [M+H]⁺ = 614.1066, C₂₇H₂₉BrN₅O₅S requires 614.1068

### Example 4 ― Preparation of 2-f4-({[6-{2-[(5-bromo-2-pyrimidinyl)oxy]ethoxy}-5-(4-methylphenyl)-4-pyrimidinyl]amino}sulfonyl)phenyl]-2-methylpropanoic acid

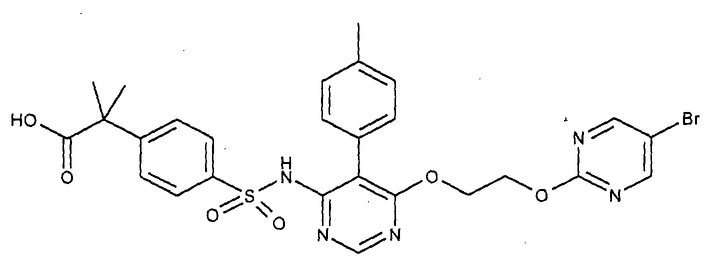

*Streptomyces rimosus* subsp. *rimosus* ATCC10970 maintained on a quarter strength ATCC172 agar slope was inoculated as a loopful of spores into a 300 ml Erlenmeyer flask containing 50ml of AS-7H inoculum medium. This was allowed to incubate for 2 days at 28°C, 200rpm on an Infors Multitron™ Shaker with 1" throw. 2mls of this inoculum medium was then transferred to each of six 300ml Erlenmeyer flask containing 50ml of AP-5H production medium and incubated under the same conditions for a further 24 hours. At this point 5mg of N-[6-{2-[(5-bromo-2-pyrimidinyl)oxy]ethoxy}-5-(4-methylphenyl)-4-pyrimidinyl]-4-(*tert*-butyl)benzenesulfonamide dissolved in 0.5ml of dimethylsulfoxide (EP 0658548B1) was added to each of the flasks and the fermentation allowed to continue under the same conditions for a further 3 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm).
Using a gradient mobile phase of 60:40 to 5:95 water:acetonitrile from 2 to 14 minutes at a flow rate of 20ml/min, the product was eluted at 7.0min. The product fractions were concentrated under reduced pressure to yield a colourless amorphous solid (1.3mg).

HPLC retention time ― Method A; 12.4 minutes.

δH (300MHz, CDCl₃)(selected nmr data) 8.55 (2H, s); 8.40 (1H, s); 7.85 (2H, d); 7.45 (2H, d) 7.20 (2H, d); 6.95(2H, d); 4.65 (2H, m); 4.55 (2H, m); 2.40 (3H, s); 1.60 (6H, s)
m/z (ESI) [M+H]⁺ = 630.0833 C₂₇H₂₆BrN₅O₆S requires 630.1980

### Example 5 ― Preparation of N-[6-{2-[(5-bromo-2-pyrimidinyl)oxy]ethoxy}-5-(4-methylphenyl)-4-pyrimidinyl]-4-(2-hydroxy-1,1-dimethylethyl)benzenesulfonamide

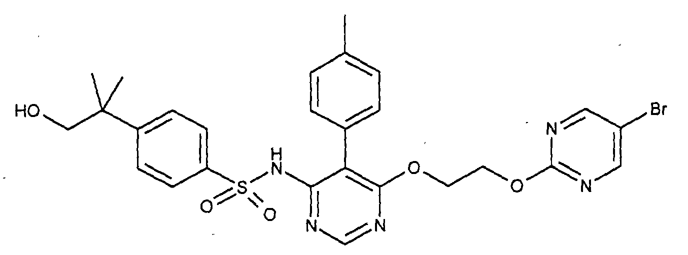

*Syncephalastrum racemosum* ATCC18192 maintained on a potato dextrose agar slope was inoculated as a loopful of spores into a 300 ml Erlenmeyer flask containing 50ml of Tomato medium. This was allowed to incubate for 2 days at 28°C, 200rpm on an Infors Multitron™ Shaker with 1" throw. At this point 3 mg of N-[6-{2-[(5-bromo-2-pyrimidinyl)oxy]ethoxy}-5-(4-methylphenyl)-4-pyrimidinyl]-4-(*tert*-butyl)benzenesulfonamide (EP 0658548B1) dissolved in 0.5ml of dimethylsulfoxide was added to the flask and the fermentation allowed to continue under the same conditions for a further 24 hours. The flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm). Using a gradient mobile phase of 50:50 to 5:95 water:acetonitrile from 2 to 14 minutes at a flow rate of 20ml/min, the product was eluted at 5.1min. The product fractions were concentrated under reduced pressure to yield the title compound as a colourless amorphous white solid (1.3mg).

HPLC retention time ― Method A; 12.5 minutes.

δH (300MHz, CDCl₃)(selected nmr data) 8.45 (2H, s); 8.40 (1H, s); 8.05 (2H, d); 7.55 (2H, d); 7.20 (2H, d); 7.10 (2H, d); 4.65 (2H, m); 4.60 (2H, m); 3.65 (2H, s); 2.40 (3H, s); 1.35 (6H, s)
m/z (ESI, FTMS) [M+H]⁺ 614.1059, C₂₇H₂₉BrN₅O₅S requires 614.1068

### Example 6 ― Preparation of the N-[4-(2-hydroxyethoxy)-5-(2-methoxyphenoxy)-2-(2-pyrimidinyl)pyrimidin-6-yl]4-(2-hydroxy-1,1-dimethylethyl)benzenesulphonamide.

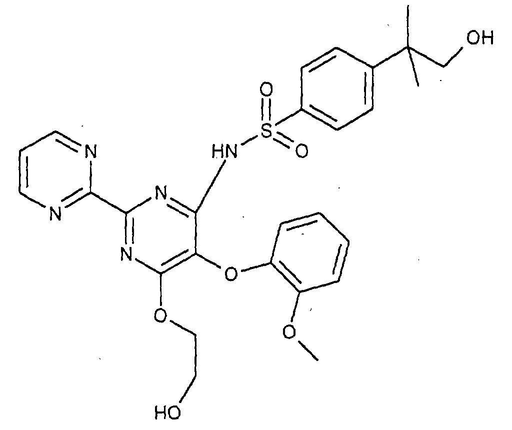

*Streptomyces rimosus* subsp. *rimosus* ATCC10970 maintained on a quarter strength ATCC172 agar slope was inoculated as a loopful of spores into a 300 ml Erlenmeyer flask containing 50ml of AS-7H inoculum medium. This was allowed to incubate for 2 days at 28°C, 200rpm on an Infors Multitron™ Shaker with 1" throw. 2mls of this inoculum medium was then transferred to one 300ml Erlenmeyer flask containing 50ml of AP-5H production medium and incubated under the same conditions for a further 24 hours. At this point 1mg of 4-*tert* butyl-N-[4-(2-hydroxyethoxy)-5-(2-methoxyphenoxy)-2-(2-pyrimidinyl)pyrimidin-6-yl]benzenesulphonamide (CA 2071193) dissolved in 0.5ml of dimethylsulfoxide was added to each of the flasks and the fermentation allowed to continue under the same conditions for a further 24 hours. The flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm). Using a gradient mobile phase of 60:40 to 5:95 water:acetonitrile from 2 to 14 minutes at a flow rate of 20ml/min, the product was eluted at 10.9 min. The product fractions were concentrated under reduced pressure to yield the title compound as a colourless amorphous solid (0.5mg).
HPLC retention time ― Method B; 4.7 minutes.

δH (400MHz, CDCl₃)(selected nmr data) 9.20 (2H, br s); 8.35 (2H, m); 7.65 (1H, br s); 7.45 (2H, m); 7.10 (2H, m); 6.95 (1H, m); 6.90 (1H, m); 4.65 (2H, m); 3.90 (3H, s); 3.85 (2H, m); 3.60 (2H, s); 1.30 (6H, s)
m/z (ESI, FTMS) [M+H]⁺ = 568.1834, C₂₇H₃₀N₅O₇S requires 568.1860

### Example 7 ― Preparation of 4-hydroxy-N-[6-methoxy-5-(2-methoxyphenoxy)-2-(2-pyrimidinyl)-pyrimidin-4-yl]-3,3-dimethyl-1-butanesulfonamide

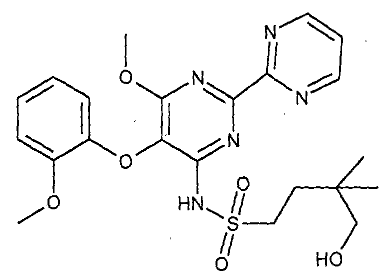

*Streptomyces rimosus* subsp. *rimosus* ATCC10970 maintained on a quarter strength ATCC172 agar slope was inoculated as a loopful of spores into a 300 ml Erlenmeyer flask containing 50ml of AS-7H inoculum medium. This was allowed to incubate for 2 days at 28°C, 200rpm on an Infors Multitron™ Shaker with 1" throw. 2mls of this inoculum medium was then transferred to twenty six 300ml Erlenmeyer flask containing 50ml of AP-5H production medium and incubated under the same conditions for a further 24 hours. At this point 4.5mg of N-[6-methoxy-5-(2-methoxyphenoxy)-2-(2-pyrimidinyl)-4-pyrimidinyl]-3,3-dimethyl-1-butanesulfonamide (Preparation 1) dissolved in 0.5ml of methanol was added to each of the flasks and the fermentation allowed to continue under the same conditions for a further 20 hours. The flask was then extracted with 200mls of ethyl acetate and the combined ethyl acetate solubles concentrated to dryness to give a gum solid, 430mg.

The crude extract was purified by preparative reversed phase HPLC with a Phenomenex Magellen™ 5µ C18 column (150mm x 21.2mm) in four injections.

Using a gradient mobile phase of 85:15 to 15:85 water/methanol from 0 to 25 minutes at a flow rate of 20ml/min, the product was eluted at 15.0 minutes. The product fractions were concentrated under reduced pressure to yield the title compound as a colourless amorphous solid (42.4 mg).
HPLC retention time ― Method A; 9.3 minutes.

δH (300MHz, CD₃OD) 8.90 (2H, d); 7.50 (1H, t); 7.00 (1H, d); 6.90 (1H, t); 6.75 (1H, t); 6.60 (1H, d); 3.95 (3H, s); 3.90 (3H, s); 3.35 (2H, m); 3.15 (2H, s); 1.60 (2H, m); 0.75 (6H, s)
m/z (ESI, FTMS) [M+H]⁺ = 490.1745, C₂₂H₂₈N₅O₆S requires 490.1755
νₘₐₓ (PE) 3406, 2920, 2850, 1582, 1563, 1501, 1473, 1426, 1386, 1132

### Example 8 ― Preparation of 5-hydroxy-N-[6-methoxy-5-(2-methoxyphenoxy)-2-(2-pyrimidinyl)-4-pyrimidinyl]-4,4-dimethyl-1-pentanesulfonamide

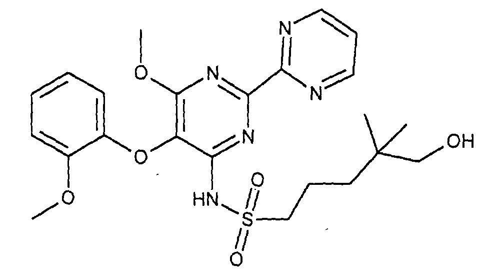

*Streptomyces rimosus* subsp. *rimosus* ATCC10970 maintained on a quarter strength ATCC172 agar slope was inoculated as a loopful of spores into a 300 ml Erlenmeyer flask containing 50ml of AS-7H inoculum medium. This was allowed to incubate for 2 days at 28°C, 200rpm on an Infors Multitron™ Shaker with 1" throw. 2mls of this inoculum medium was then transferred to twenty 300ml Erlenmeyer flask containing 50ml of AP-5H production medium and incubated under the same conditions for a further 24 hours. At this point 4.5mg of *N*-[6-methoxy-5-(2-methoxyphenoxy)-2-(2-pyrimidinyl)-4-pyrimidinyl]-4,4-dimethyl-1-pentanesulfonamide (Preparation 3) dissolved in 0.5ml of methanol was added to each of the flasks and the fermentation allowed to continue under the same conditions for a further 20 hours. The flask was then extracted with 200mls of ethyl acetate and the combined ethyl acetate solubles concentrated to dryness to give a gum solid, 200mg.

The crude extract was purified by preparative reversed phase HPLC with a Phenomenex Magellen™ 5µ C18 column (150mm x 21.2mm) in four injections. Using a gradient mobile phase of 90:10 to 40:60 water:acetonitrile from 3 to 20 minutes at a flow rate of 20ml/min, the product was eluted at 17.2 minutes. The product fractions were concentrated under reduced pressure to yield the title compound as a colourless amorphous solid (46.7 mg).
HPLC retention time ― Method A; 11.3 minutes.

δH (300MHz, CDCl₃)(selected nmr data) 8.95 (2H, d); 7.40 (1H, m); 7.05 (2H, m); 6.95 (1H, m); 6.85 (1H, m); 4.15 (3H, s); 3.95 (3H, s); 3.85 (2H, m); 3.25 (2H, s); 1.80 (2H, m); 1.40 (2H, m); 0.80 (6H, s)
m/z (ESI) [M+H]⁺ = 504.1927, C₂₃H₃₀N₅O₆S requires 504.1917
m/z (ESI) [M+Na]⁺ = 526.1749, C₂₃H₂₉N₅O₆SNa requires 526.1736
νₘₐₓ (PE) 3390, 3219, 2955, 1583, 1558, 1503, 1388, 1336, 1253, 1092, 875, 748

### Example 9 ― Preparation of 4-(2-hydroxy-1,1-dimethylethyl)-N-[6-methoxy-5-(2-methoxyphenoxy)-2-(2-pyrimidinyl)-4-pyrimidin-4-yl]benzenesulfonamide

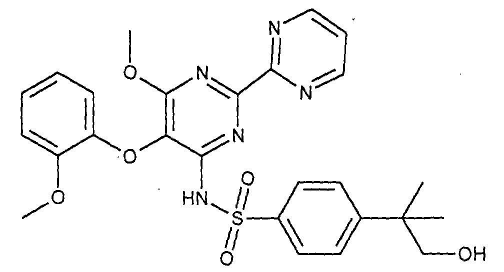

*Streptomyces rimosus* subsp. *rimosus* ATCC10970 maintained on a quarter strength ATCC172 agar slope was inoculated as a loopful of spores into a 300 ml Erlenmeyer flask containing 50ml of AS-7H inoculum medium. This was allowed to incubate for 2 days at 28°C, 200rpm on an Infors Multitron™ Shaker with 1" throw. 2mls of this inoculum medium was then transferred to twenty 300ml Erlenmeyer flask containing 50ml of AP-5H production medium and incubated under the same conditions for a further 24 hours. At this point 15mg of 4-*tert*-butyl-*N*-[6-methoxy-5-(2-methoxyphenoxy)-2-(2-pyrimidinyl)-4-pyrimidinyl]benzenesulfonamide (Preparation 7) dissolved in 0.5ml of methanol was added to each of the flasks and the fermentation allowed to continue under the same conditions for a further 20 hours.

The flask was then extracted with 200mls of ethyl acetate and the combined ethyl acetate extracts concentrated to dryness to give a gum solid, 1100mg.

The crude extract was purified by preparative reversed phase HPLC with a Phenomenex Magellen™ 5µ C18 column (150mm x 21.2mm) in five injections. Using a gradient mobile phase of 90:10 to 40:60 water:acetonitrile from 3 to 20 minutes at a flow rate of 20ml/min, the product was eluted at 19.0 minutes. The product fractions were concentrated under reduced pressure to yield the title compound as a colourless amorphous solid (84.4 mg).
HPLC retention time ― Method A; 4.9 minutes.

δH (300MHz, CDCl₃) 8.85 (1H, br s); 8.35 (2H, br s); 7.40 (3H, m); 7.10 (1H, m); 6.95 (2H, m); 6.80 (1H, m); 4.10 (3H, s); 3.95 (3H, s); 3.55 (2H, s); 1.80 (1H, br s); 1.30 (6H, s)
m/z (ESI, FTMS) [M+H]⁺ = 538.1748, C₂₆H₂₈N₅O₆S requires 538.1755
m/z (ESI, FTMS) [M+Na]⁺ = 560.1585, C₂₆H₂₇N₅O₆SNa requires 560.1574
νₘₐₓ (PE) 3396, 2964, 1580, 1560, 1501, 1389, 1253, 1169, 1083, 865

### Example 10 ― Preparation and Isolation of N-[3-(2-ethoxyethoxy)-1-methyl-4-(4-methylphenyl)-1H-pyrazol-5-yl]-4-(2-hydroxy-1,1-dimethylethyl)benzenesulfonamide

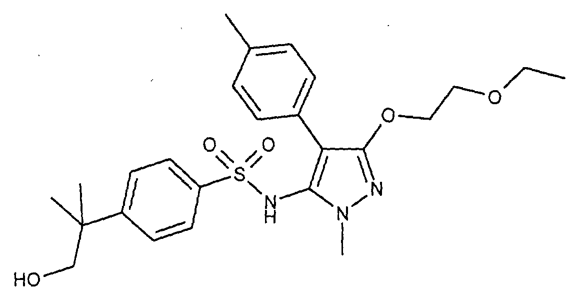

*Amycolata autotrophica* ATCC35203 maintained on a quarter strength ATCC172 agar slope was inoculated as a loopful of spores into five 300 ml Erlenmeyer flasks each containing 50ml of MY inoculum medium. This was allowed to incubate for 2 days at 28°C, 200rpm on an Infors Multitron™ Shaker with 1" throw. Two mls of this inoculum was then transferred to each of sixty 300ml Erlenmeyer flask containing 50ml of MY production medium and incubated under the same conditions for a further 24 hours. At this point 20mg of 4-(*tert*-butyl)-*N*-[3-(2-ethoxyethoxy)-1-methyl-4-(4-methylphenyl)-1H-pyrazol-5-yl]benzenesulfonamide (Preparation 8) dissolved in 0.5ml of methanol was added to each of the flasks and the fermentation allowed to continue under the same conditions for a further 6 hours. Each flask was then extracted with 200mls of ethyl acetate and the combined ethyl acetate solubles concentrated to dryness to give a gum solid, 2.0 g.

The crude extract was purified by preparative reversed phase HPLC with a Phenomenex Magellen™ 5µ C18 column (150mm x 21.2mm) in five injections. Using a gradient mobile phase of 35:65 to 20:80 water:methanol from 1.5 to 20 minutes at a flow rate of 20ml/min, the product was eluted at 9.1 minutes. The product fractions were concentrated under reduced pressure to yield the title compound as a colourless amorphous solid (3.0 mg).
HPLC retention time ― Method A; 10 minutes.

δH (300MHz, CDCl₃) 7.40 (2H, d), 7.15 (2H, d); 6.90 (4H, m); 4.35 (2H, m); 3.85 (3H, s); 3.85 (2H, m); 3.55 (2H, q); 3.50 (2H, s); 2.25 (3H, s); 1.25 (6H, s) 1.20 (3H, t)
m/z (ESI) [M+H]⁺ = 488.2218, C₂₅H₃₄N₃O₅S requires 488.2219
m/z (ESI) [M+Na]⁺ = 510.2026, C₂₅H₃₃N₃O₅SNa requires 510.2039
νₘₐₓ (PE) 3352, 2920, 1572, 1519, 1428, 1328, 1167

### Example 11 ― Preparation of N-(4-(1,3-benzodioxol-5-yl)-3-{2-[(5-chloro-2-pyrimidinyl)oxy]ethoxy}-1-methyl-1H-pyrazol-5-yl)-4-(2-hydroxy-1,1-dimethylethyl)benzenesulfonamide

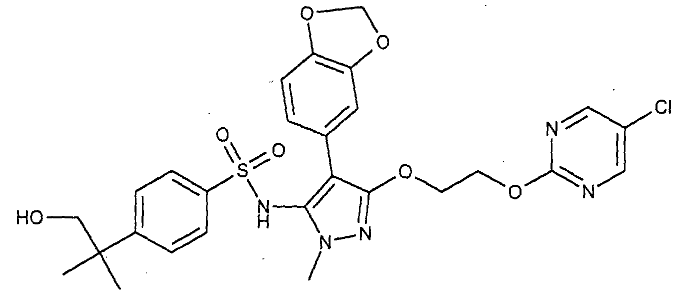

*Amycolata autotrophica* ATCC35203 maintained on a quarter strength ATCC172 agar slope was inoculated as a loopful of spores into a 300 ml Erlenmeyer flasks each containing 50ml of MY inoculum medium. This was allowed to incubate for 2 days at 28°C, 200rpm on an Infors Multitron™ Shaker with 1" throw. Two mls of this inoculum was then transferred to each of nine 300ml Erlenmeyer flask containing 50ml of MY production medium and incubated under the same conditions for a further 24 hours. At this point 5mg of *N*-(4-(1,3-benzodioxol-5-yl)-3-{2-[(5-chloro-2-pyrimidinyl)oxy]ethoxy}-1-methyl-1*H*-pyrazol-5-yl)-4-(*tert*-butyl)benzenesulfonamide (GB Patent Application No 9917858.4) dissolved in 0.5ml of methanol was added to each of the flasks and the fermentation allowed to continue under the same conditions for a further 96 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm) in seven injections. Using a gradient mobile phase of 35:65 to 20:80 water:methanol from 1.5 to 20 minutes at a flow rate of 20ml/min, the product was eluted at 8.4 minutes. The product fractions were concentrated under reduced pressure to yield the title compound as a colourless amorphous solid (10.0 mg).
HPLC retention time ― Method A; 8 minutes.

δH (300MHz, CDCl₃) 8.40 (2H, s); 7.40 (2H, d); 7.20 (2H, d); 6.45 (2H, s); 6.40 (1H, s); 5.85 (2H, s); 4.70 (2H, m); 4.50 (2H, m); 3.85 (3H, s); 3.55 (2H, s); 1.25 (6H, s) m/z (ESI) [M+H]⁺ = 602.1474, C₂₇H₂₉ClN₅O₇S requires 602.1476
m/z (ESI) [M+Na]⁺ = 624.1273, C₂₇H₂₈ClN₅O₇SNa requires 624.1296
νₘₐₓ (NaCI) 3384, 2964, 1580, 1551, 1486, 1428, 1325, 1231, 1163, 1107, 1041, 936

### Example 12 ― Preparation of N-[4-(1,3-benzodioxol-5-yl)-3-(2-hydroxyethoxy)-1-methyl-1H-pyrazol-5-yl]-4-(2-hydroxy-1,1-dimethylethyl)benzenesulfonamide

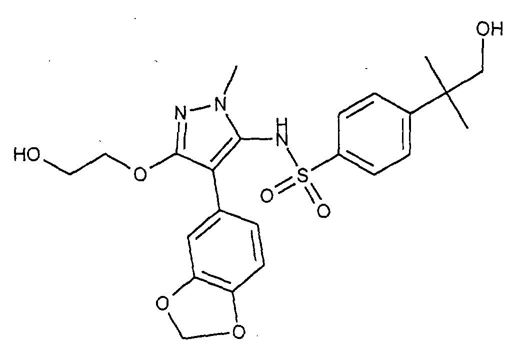

*Amycolata autotrophica* ATCC35203 maintained on a quarter strength ATCC172 agar slope was inoculated as a loopful of spores into a 300 ml Erlenmeyer flasks each containing 50ml of MY inoculum medium. This was allowed to incubate for 2 days at 28°C, 200rpm on an Infors Multitron™ Shaker with 1" throw. Two mls of this inoculum was then transferred to each of twenty 300ml Erlenmeyer flask containing 50ml of MY production medium and incubated under the same conditions for a further 24 hours. At this point 5mg of N-[4-(1,3-benzodioxol-5-yl)-3-(2-hydroxyethoxy)-1-methyl-1*H*-pyrazol-5-yl]-4-(*tert*-butyl)benzenesulfonamide (GB Patent Application No 9917858.4) dissolved in 0.5ml of methanol was added to each of the flasks and the fermentation allowed to continue under the same conditions for a further 96 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm) in two injections. Using a gradient mobile phase of 85:15 to 15:85 water:methanol from 0 to 25 minutes at a flow rate of 20ml/min, the product was eluted at 14.4 minutes. The product fractions were concentrated under reduced pressure to yield the title compound as a colourless amorphous solid (19.8 mg).
HPLC retention time ― Method A; 3.8 minutes.

δH (300MHz, CD₃OD) 7.45 (2H, d); 7.20 (2H, d); 6.80 (1H, s); 6.75 (1H, d); 6.45 (1H, d); 5.85 (2H, s); 4.20 (2H, m); 3,80 (2H, m); 3.70 (3H, s); 3.45 (2H, s); 1.25 (6H, s) m/z (ESI) [M+H]⁺ = 490.1669, C₂₃H₂₈N₃O₇S requires 490.1648
m/z (ESI) [M+Na]⁺ = 512,1467, C₂₃H₂₇N₃O₇SNa requires 512.1467
νₘₐₓ(PE) 3310, 2923, 1596, 1502, 1336, 1231, 1164, 1106, 1040, 934

### Example 13 ― Preparation of N-(4-(1,3-benzodioxol-5-yl)-3-{2-[(5-chloro-2-pyrimidinyl)oxy]ethoxy}-1-methyl-1H-pyrazol-5-yl)-5-(2-hydroxy-1-methylethyl)-2-pyridinesulfonamide

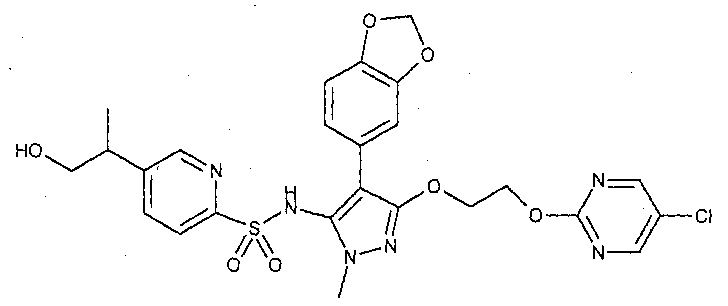

*Streptomyces rimosus* subsp. *rimosus* ATCC10970 maintained on a quarter strength ATCC172 agar slope was inoculated as a loopful of spores into a 300 ml Erlenmeyer flask containing 50ml of AS-7H inoculum medium. This was allowed to incubate for 2 days at 28°C, 200rpm on an Infors Multitron™ Shaker with 1" throw. 2mls of this inoculum medium was then transferred to each of twenty 300ml Erlenmeyer flask containing 50ml of AP-5H production medium and incubated under the same conditions for a further 24 hours. At this point 5mg of *N*-(4-(1,3-benzodioxol-5-yl)-3-{2-[(5-chloro-2-pyrimidinyl)oxy]ethoxy}-1-methyl-1*H*-pyrazol-5-yl)-5-isopropyl-2-pyridinesulfonamide (Preparation12) dissolved in 0.5ml of methanol was added to each of the flasks and the fermentation allowed to continue under the same conditions for a further 96 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm) in one injection. Using a gradient mobile phase of 85:15 to 15:85 water/methanol from 0 to 25 minutes at a flow rate of 20ml/min, the product was eluted at 16.0 minutes. The fractions containing product were further purified using a Phenomenex Lichrosphere™ 5µ DIOL column (250mm x 21.2mm) in one injection. Using an isocratic mobile phase of 40:60 isopropanol:dichloromethane, the product was eluted at 7.2 minutes. The product fractions were concentrated under reduced pressure to yield the title compound as a colourless amorphous solid (8.4 mg).
HPLC retention time - Method A; 5.5 minutes.

δH (400MHz, CD₃OD) 8.50 (2H, s); 8.20 (1H, s); 7.55 (2H, m); 6.60 (2H, m); 6.45 (1H, d); 5.85 (2H, s); 4.70 (2H, m); 4.50 (2H, m); 3.75 (3H, s); 3.55 (2H, d); 2.85 (1H, m); 1.15 (3H, d)
m/z (ESI) [M+H]⁺ = 589.1267, C₂₅H₂₆ClN₆O₇S requires 589.1272
m/z (ESI) [M+Na]⁺ = 611.1101, C₂₅H₂₅ClN₆O₇SNa requires 611.1092
νₘₐₓ (PE) 3239, 2949, 1580, 1427, 1326, 1232, 1176, 1041

### Example 14 ― Preparation of 2-[4-({[3-(2-methoxyethoxy)-1-methyl-4-(4-methylphenyl)1H-pyrazol-5-yl]amino}sulfonyl)phenyl]-2-methylpropanoic acid

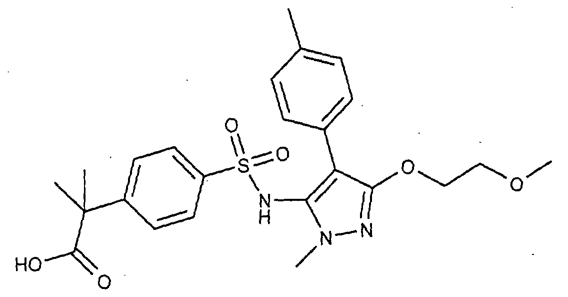

*Amycolata autotrophica* ATCC35203 maintained on a quarter strength ATCC172 agar slope was inoculated as a loopful of spores into a 300 ml Erlenmeyer flasks each containing 50ml of MY inoculum medium. This was allowed to incubate for 2 days at 28°C, 200rpm on an Infors Multitron™ Shaker with 1" throw. Two mls of this inoculum was then transferred to each of six 300ml Erlenmeyer flask containing 50ml of MY production medium and incubated under the same conditions for a further 24 hours. At this point 5mg of 4-(*tert*-butyl)-*N*-[3-(2-methoxyethoxy)-1-methyl-4-(4-methylphenyl)-1*H*-pyrazol-5-yl]benzenesulfonamide (Preparation 17) dissolved in 0.5ml of methanol was added to each of the flasks and the fermentation allowed to continue under the same conditions for a further 96 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm) in one injection. Using a gradient mobile phase of 85:15 to 15:85 water:methanol from 0 to 25 minutes at a flow rate of 20ml/min, the product was eluted at 17.0 minutes. The product fractions were concentrated under reduced pressure to yield the title compound as a colourless amorphous solid (4.0 mg).
HPLC retention time ― Method A; 7.4 minutes.

δH (300MHz, CDCl₃: CD₃OD 3:1) 7.30 (4H, m); 7.05 (2H, d); 6.80 (2H, d); 4.15 (2H, m); 3.60 (2H, m); 3.35 (3H, s); 3.30 (3H, s); 2.15 (3H, s); 1.35 (6H, s)
m/z (ESI, FTMS) [M+H]⁺ = 488.1833, C₂₄H₃₀N₃O₆S requires 488.1850
νₘₐₓ (PE) 3373, 2968, 1564, 1483, 1399, 1358, 1171, 1134, 1099

### Example 15 ― Preparation of 2-[4-({[3-(2-ethoxyethoxy)-1-methyl-4-(4-methylphenyl)-1H-pyrazol-5-yl]amino}sulfonyl)phenyl]-2-methylpropanoic acid

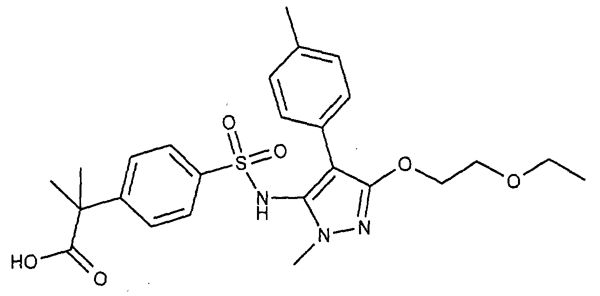

*Amycolata autotrophica* ATCC35203 maintained on a quarter strength ATCC172 agar slope was inoculated as a loopful of spores into a 300 ml Erlenmeyer flasks each containing 50ml of MY inoculum medium. This was allowed to incubate for 2 days at 28°C, 200rpm on an Infors Multitron™ Shaker with 1" throw. Two mls of this inoculum was then transferred to each of six 300ml Erlenmeyer flask containing 50ml of MY production medium and incubated under the same conditions for a further 24 hours. At this point 5mg of 4-(*tert*-butyl)-N-[3-(2-ethoxyethoxy)-1-methyl-4-(4-methylphenyl)-1*H*-pyrazol-5-yl]benzenesulfonamide (Preparation 8) dissolved in 0.5ml of methanol was added to each of the flasks and the fermentation allowed to continue under the same conditions for a further 96 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm) in one injection. Using a gradient mobile phase of 85:15 to 15:85 water:methanol from 0 to 25 minutes at a flow rate of 20ml/min, the product was eluted at 16.5 minutes. The product fractions were concentrated under reduced pressure to yield the title compound as a colourless amorphous solid (1.5 mg).
HPLC retention time ― Method A; 9.3 minutes.

δH (300MHz, CDCl₃: CD₃OD 3:1) 7.30 (4H, m); 7.05 (2H, d); 6.75 (2H, d); 4.10 (2H, m); 3.60 (2H, m); 3.45 (2H, q); 3.35 (3H, s); 2.10 (3H, s); 1.30 (6H, s); 1.05 (3H, t)
m/z (ESI, FTMS) [M+H]⁺ = 502.1995, C₂₅H₃₂N₃O₆S requires 502.2006
m/z (ESI, FTMS) [M+Na]⁺ = 524.1811, C₂₅H₃₁N₃O₆SNa requires 524.1825

### Example 16 ― Preparation of N-(4-(1,3-benzodioxol-5-yl)-3-{2-[(5-bromo-2-pyrimidinyl)oxy]ethoxy}-5-isoxazolyl)-4-(2-hydroxy-1,1-dimethylethyl)benzenesulfonamide

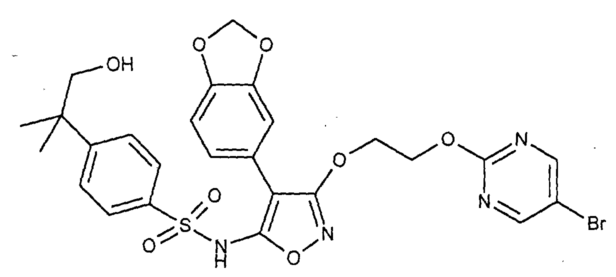

*Amycolata autotrophica* ATCC35203 maintained on a quarter strength ATCC172 agar slope was inoculated as a loopful of spores into a 300 ml Erlenmeyer flasks each containing 50ml of of MY inoculum medium. This was allowed to incubate for 2 days at 28°C, 200rpm on an Infors Multitron™ Shaker with 1" throw. Two mls of this inoculum was then transferred to each of twenty 300ml Erlenmeyer flask containing 50ml of MY production medium and incubated under the same conditions for a further 24 hours. At this point 5mg of *N*-(4-(1,3-benzodioxol-5-yl)-3-{2-[(5-bromo-2-pyrimidinyl)oxy]ethoxy}-5-isoxazolyl)-4-(*tert*-butyl)benzenesulfonamide (Preparation 20) dissolved in 0.5ml of methanol was added to each of the flasks and the fermentation allowed to continue under the same conditions for a further 144 hours. Each flask was then extracted with 200mls of ethyl acetate and the combined ethyl acetate solubles concentrated to dryness to give a gum solid.

The crude extract was purified by preparative reversed phase HPLC with a Phenomenex Magellen™ 5µ C18 column (150mm x 21.2mm) in two injections. Using a gradient mobile phase of 35:65 to 20:80 water:methanol from 1.5 to 29 minutes at a flow rate of 20ml/min, the product was eluted at 4.1 minutes. The product fractions were purified again on the same column in one injection. Using a gradient mobile phase of 85% to 15% water/methanol from 0 to 25 minutes at a flow rate of 20ml/min, the product was eluted at 14.9 minutes. The product fractions were concentrated under reduced pressure to yield the title compound as a colourless amorphous solid (2.0 mg).
HPLC retention time ― Method A; 8.6 minutes.

δH (300MHz, CDCl₃) 8.40 (2H, s); 7.70 (2H, d); 7.10 (2H, d); 7.05 (2H, m); 6.55 (1H, d); 5.75 (2H, s); 4.55 (2H, m); 4.40 (2H, m); 3.35 (2H, s); 1.05 (6H, s)
m/z (ESI) [M+H]⁺ = 633.0655, C₂₆H₂₆BrN₄O₈S requires 633.0655
m/z (ESI) [M+Na]⁺ = 655.0481, C₂₆H₂₅BrN₄O₈SNa requires 655.0474
νₘₐₓ (PE) 3373, 2968, 1564, 1483, 1399, 1358, 1171, 1134, 1099

### Preparations

### Preparation 1 ― Preparation of N-[6-methoxy-5-(2-methoxyphenoxy)-2-(2-pyrimidinyl)-4-pyrimidinyl]-3,3-dimethyl-1-butanesulfonamide

Sodium hydride (80% oil dispersion 132mg) was cautiously added to a suspension of *N*-[6-chloro-5-(2-methoxyphenoxy)-2-(2-pyrimidinyl)-4-pyrimidinyl]-3,3-dimethyl-1-butanesulfonamide (Preparation 2)(420mg) in dry methanol (12ml) divided equally between two 10ml Wheaton reacti-vials™, and the resulting mixtures stirred at 100°C for 3 hours. The reactions were then combined and partitioned between ethyl acetate (40ml) and 2N aqueous hydrochloric acid (20ml) and the organic phase separated. The aqueous was extracted with ethyl acetate (20ml) and the organic phases combined, washed (water 2x20ml), dried (Na₂SO₄) and evaporated under reduced pressure. Column chromatography on silica eluting with a solvent gradient of dichloromethane: methanol 99:1 changing to 97:3 gave N-[6-methoxy-5-(2-methoxyphenoxy)-2-(2-pyrimidinyl)-4-pyrimidinyl]-3,3-dimethyl-1-butanesulfonamide as an off-white coloured foam (265mg).

δH (400MHz, *d*_{*6*}DMSO + 2dps TFA-D) 8.95 (2H, m); 7.60 (1H, m); 7.10 (1H, d); 7.00 (1H, t); 6.80 (1H, t); 6.65 (1H, d); 3.90 (2H, m); 3.90 (3H, s); 3.80 (3H, s); 1.60 (2H, m); 0.85 (9H, s)
m/z (TSPI) [M+H]⁺ = 474.0, C₂₂H₂₈N₅O₅S requires 474.2
Analysis: Found C, 51.32; H, 5.51; N, 13.17: C₂₂H₂₇N₅O₅S. 0.6 CH₂Cl₂ requires C, 51.75; H, 5.42; N, 13.35.

### Preparation 2 ― Preparation of N-[6-chloro-5-(2-methoxyphenoxy)-2-(2-pyrimidinyl)-4-pyrimidinyl]-3,3-dimethyl-1-butanesulfonamide

Potassium *tert*-butoxide (340mg) was added to 3,3-dimethylbutane-1-sulphonic acid amide (J.Org. Chem., 1956, **21,** 385) (500mg) in dry tetrahydrofuran (10ml) at room temperature under nitrogen. The resulting mixture was stirred for I hour and then concentrated under reduced pressure. The residual potassium salt was re-evaporated from dry toluene, oven dried at 40°C under vacuum, and used directly.

3,3-dimethylbutane-1-sulphonic acid amide potassium salt (616mg) was added to a solution of 4,6-dichloro-5-(2-methoxyphenoxy)-2-(pyrimidin-2-yl)-pyrimidine (Can. Pat. Appl. 2071193) (353mg) in dry dimethyl sulphoxide (10ml) and the resulting mixture stirred at 120°C for 6 hours under nitrogen. The reaction mixture was allowed to cool and then partitioned between ethyl acetate (30ml) and 2N aqueous hydrochloric acid (30ml) and the organic phase separated. The aqueous was extracted with ethyl acetate (30ml) and the organic phases combined, washed (water 2x20ml), dried (Na₂SO₄) and evaporated under reduced pressure. Column chromatography on silica eluting with a solvent gradient of pentane: ethyl acetate 50:50 changing to 0:100 and finally ethyl acetate: methanol 98:2 gave *N*-[6-chloro-5-(2-methoxyphenoxy)-2-(2-pyrimidinyl)-4-pyrimidinyl]-3,3-dimethyl-1-butanesulfonamide as an off-white solid (440mg).

δH (300MHz, *d*_{*6*}DMSO) 9.00 (2H, d); 7.65 (1H, t); 7.10 (2H, m); 6.85 (2H, m); 6.75 (1H, bs); 3.95 (2H, m); 3.80 (3H, s); 1.55 (2H, m); 0.95 (9H, s)
m/z (TSPI) [M+H]⁺ = 478.0, C₂₁H₂₅ClN₅O₄S requires 478.1

### Preparation 3 ― Preparation of N-[6-methoxy-5-(2-methoxyphenoxy)-2-(2-pyrimidinyl)-4-pyrimidinyl]-4,4-dimethyl-1-pentanesulfonamide

Sodium hydride (60% oil dispersion 354mg) was cautiously added to a suspension of *N*-[6-chloro-5-(2-methoxyphenoxy)-2-(2-pyrimidinyl)-4-pyrimidinyl]-4,4-dimethyl-1-pentanesulfonamide (580mg) in dry methanol (5ml) in a 10ml Wheaton reacti-vial™, and the resulting mixture stirred at 105°C for 4 hours. The reaction was then partitioned between ethyl acetate (20ml) and 2N aqueous hydrochloric acid (10ml) and the organic phase separated. The aqueous was extracted with ethyl acetate (20ml) and the organic phases combined, washed (water 2x20ml), dried (Na₂SO₄) and evaporated under reduced pressure. Column chromatography on silica eluting with a solvent gradient of dichloromethane: methanol 100:0 changing to 98.5:1.5 gave N-[6-methoxy-5-(2-methoxyphenoxy)-2-(2-pyrimidinyl)-4-pyrimidinyl]-4,4-dimethyl-1-pentanesulfonamide as an off-white coloured solid (189mg).

δH (400MHz, CDCl₃) 8.95 (2H, d); 8.40 (1H, br s); 7.35 (1H, t); 7.10 (2H, m); 7.00 (1H, d); 6.90 (1H, t); 4.15 (3H, s); 4.00 (3H, s); 3.85 (2H, t); 1.85 (2H, m); 1.30 (2H, m); 0.85 (9H, s)
m/z (TSPI) [M+H]⁺ = 488.0, C₂₃H₃₀N₅O₅S requires 488.2
Analysis: Found C, 55.84; H, 6.01; N, 14.06: C₂₃H₂₉N₅O₅S. 0.5 H₂O requires C, 55.63; H, 6.09; N, 14.10.

### Preparation 4 ― Preparation of N-[6-chloro-5-(2-methoxyphenoxy)-2-(2-pyrimidinyl)-4-pyrimidinyl]-4,4-dimethyl-1-pentanesulfonamide

Caesium carbonate (2.62g) was added to a solution of 4,4-dimethylpentane-1-sulphonic acid amide (720mg) and 4,6-dichloro-5-(2-methoxyphenoxy)-2-(pyrimidin-2-yl)-pyrimidine (Can. Pat. Appl. 2071193) (1.40g) in dry N,N-dimethyl formamide (8ml) and the resulting mixture stirred at 80°C for 4 hours under nitrogen. The reaction mixture was allowed to cool and then partitioned between ethyl acetate (40ml) and 2N aqueous hydrochloric acid (20ml). The organic phase was separated, washed (brine 2x20ml), dried (Na₂SO₄) and evaporated under reduced pressure. Column chromatography on silica eluting with a solvent gradient of dichloromethane: methanol 100:0 changing to 99:1 gave crude product which was recrystallised from ethyl acetate: pentane to give *N*-[6-chloro-5-(2-methoxyphenoxy)-2-(2-pyrimidinyl)-4-pyrimidinyl]-4,4-dimethyl-1-pentanesulfonamide as a white solid (1.08g).

δH (400MHz, CDCl₃) 8.95 (2H, d); 8.55 (1H, br s); 7.40 (1H, t); 7.20 (1H, t); 7.10 (1H, d); 7.05 (1H, d); 6.95 (1H, t); 3.95 (3H, s); 3.90 (2H, m); 1.85 (2H, m); 1.30 (2H, m); 0.85 (9H, s)
m/z (TSPI) [M+H]⁺ = 492.0, C₂₂H₂₆ClN₅O₄S

### Preparation 5 ― Preparation of 4,4-dimethylpentane sulphonic acid amide

Anhydrous ammonia gas was passed into a solution of 4,4-dimethylpentane sulphonyl chloride (1.85g) in dry tetrahydrofuran (5ml) at 5-10°C until saturation. The resulting suspension was stirred at room temperature for 18 hours and then evaporated under reduced pressure. The residue was partitioned between ethyl acetate (20ml) and 2N aqueous hydrochloric acid (20ml). The organic phase was separated, washed (brine 20ml), dried (Na₂SO₄) and evaporated. Column chromatography on silica eluting with dichloromethane: methanol 98:2 gave 4,4-dimethylpentane sulphonic acid amide as an off-white solid (0.72g).

δH (400MHz, CDCl₃) 4.50 (2H, br s); 3.05 (2H, t); 1.85 (2H, m); 1.30 (2H, t); 0.90 (9H, s)
m/z (TSPI) [M+NH₄]⁺ = 197.1, C₇H₂₁N₂O₂S requires 197.1

### Preparation 6 ― Preparation of 4,4-dimethylpentane sulphonyl chloride

Sodium sulphite (5.35g) was added to a solution of 1-bromo-4,4-dimethylpentane (DE 2040784) (1.9g) in a mixture of dioxan (15ml) and water (15ml). The resulting mixture was stirred at reflux for 15 hours and then evaporated under reduced pressure. The residue was triturated with toluene (2x30ml), re-evaporated from toluene (2x30ml) and then suspended in thionyl chloride (10ml) and 2 drops of N,N-dimethylformamide added. The mixture was stirred at reflux for 1 hour, then allowed to cool and evaporated under reduced pressure. The residue was re-evaporated from toluene (25ml) and then partitioned between ethyl acetate (25ml) and 2N aqueous hydrochloric acid (25ml), The organic phase was separated, washed (brine 25ml), dried (Na₂SO₄) and evaporated to yield 4,4-dimethylpentane sulphonyl chloride as an orange/brown coloured oil (1.9g).

δH (400MHz, CDCl₃) 3.60 (2H, t); 2.00 (2H, m); 1.30 (2H, m); 0.90 (9H, s)

### Preparation 7 ― Preparation of 4-tert-butyl-N-[6-methoxy-5-(2-methoxyphenoxy)-2-(2-pyrimidinyl)-4-pyrimidinyl]benzenesulfonamide

4-*tert-*Butyl-benzene sulphonyl chloride (286mg) was added to a stirred solution of 4-amino-6-methoxy-5-(2-methoxyphenoxy)-2-(2-pyrimidinyl)-pyrimidine (EP 713875A1) (100mg) in dry pyridine (2ml) at room temperature and the resulting mixture stirred for 36 hours. The reaction was then partitioned between ethyl acetate (20ml) and 2N aqueous hydrochloric acid (10ml) and the organic phase separated, washed (2N aqueous hydrochloric acid 2x10ml, water 2x10ml), dried (MgSO₄) and evaporated under reduced pressure. Column chromatography on silica eluting with a solvent gradient of pentane: ethyl acetate 25:75 changing to 0:100, then dichloromethane: methanol 95:5 gave 4-*tert*-butyl-N-[6-methoxy-5-(2-methoxyphenoxy)-2-(2-pyrimidinyl)-4-pyrimidinyl]benzenesulfonamide as an off-white coloured solid (5mg).

δH (400MHz, CDCl₃) 9.00 (2H, d); 8.70 (1H, br s); 8.35 (2H, d); 7.40 (3H, m); 7.10 (1H, t); 7.00 (2H, d); 6.85 (1H, t); 4.10 (3H, s); 4.00 (3H, s); 1.25 (9H, s)
m/z (ESI, FTMS) [M+H]⁺ = 522.1798, C₂₆H₂₇N₅O₅S requires 522.1806

### Preparation 8 ― Preparation of 4-(tert-butyl)-N-[3-(2-ethoxvethoxv)-1-methyl-4-(4-methylphenyl)-1H-pyrazol-5-yl)benzenesulfonamide

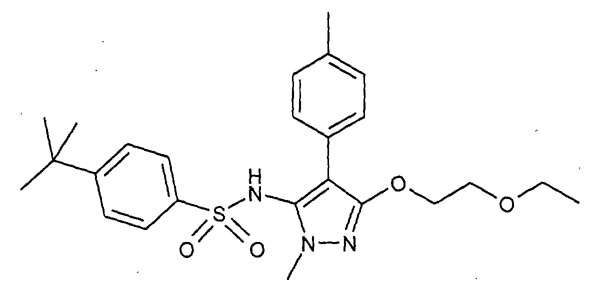

The method employed for Preparation 17 was used to prepare the title compound from 4-(*tert*-butyl)-N-{[4-(*tert*-butyl)phenyl]sulfonyl}-N-[3-(2-ethoxyethoxy)-1-methyl-4-(4-methylphenyl)-1 H-pyrazol-5-yl]benzenesulfonamide (Preparation 9)

δ_{H} (400MHz, CDCl₃) 7.36 (2H, d), 7.12 (2H, d), 6.85 (2H, d), 6.80 (2H, d), 6.58 (1H, s), 4.32 (2H, m), 3.83 (3H, s), 3.72 (2H, m), 3.52 (2H, q), 2.23 (3H, s), 1.24 (9H, s), 1.89 (3H, s).
^{m}/_{z} (ESI) [MH⁺] = 472.0, C₂₅H₃₄N₃O₄S requires 472.1

### Preparation 9 ― Preparation of 4-(tert-butyl)-N-{[4-(tert-butyl)phenyl]sulfonyl}-N-[3-(2-ethoxyethoxy)-1-methyl-4-(4-methylphenyl)-1H-pyrazol-5-yl]benzenesulfonamide

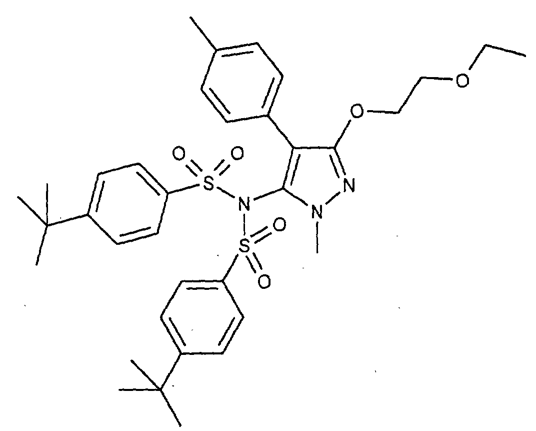

The method of Preparation 18 was used to prepare the title compound from 3-(2-ethoxyethoxy)-1-methyl-4-(4-methylphenyl)-1*H*-pyrazol-5-ylamine (Preparation 10)

δ_{H} (400MHz, CDCl₃) 7.80 (4H, d), 7.40 (6H, m), 6.85 (2H, d), 4.40 (2H, m), 3.75 (2H, m), 3.40 (2H, q), 3.15 (3H, s), 2.25 (3H, s), 1.35 (18H, s), 1.25 (3H, t).
^{m}/_{z} (TSPI) [MH⁺] = 668.0, C₃₅H₄₆N₅S₂O₆ requires 668.1

### Preparation 10 ― Preparation of 3-(2-ethoxyethoxy)-1-methyl-4-(4-methylphenyl)-1pyrazol-5-ylamine

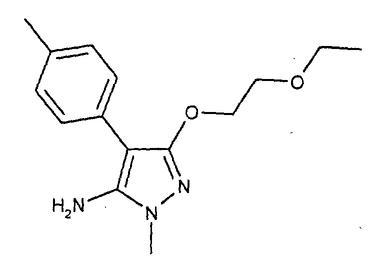

The method of Preparation 19 was used to prepare the title compound from 5-amino-1-methyl-4-(4-methylphenyl)-1*H*-pyrazol-3-ol (Preparation 11).

δ_{H} (400MHz, CDCl₃) 7.35 (2H, d), 7.20 (2H, d), 4.35 (2H, m), 3.70 (2H, m), 3.65 (2H, s), 3.55 (5H, m), 2.35 (3H, s), 1.20 (3H, t).
^{m}/_{z} (TSPI) [MH⁺] = 276.0, C₁₅H₂₂N₃O₂ requires 276.1

### Preparation 11 ― Preparation of 5-amino-1-methyl-4-(4-methylphenyl)-1H-pyrazol-3 ol

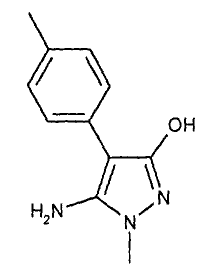

To ethyl (4-methylphenyl)cyanoacetate (*Synthesis,* **1985,** 5, 506) (22.5g) in ethanol (150ml) at room temperature was added methylhydrazine (8.8ml) dropwise over 30 minutes the mixture was heated to reflux temperature for 20hrs. The reaction was evaporated to dryness. The residue was recrystalised in ethanol to yield the title compound as a white solid (8.3g).
δ_{H} (300MHz, *d*_{*6*}DMSO) 9.40 (1H, br. s), 7.40 (2H, d), 7.05 (2H, d), 5.60 (2H, br. s), 3.10 (3H, s), 2.25 (3H, s).
*m/z* (TSPI) [MH⁺] =204.2, C₁₁H₁₄N₃O requires 204.1

### Example 12 ― Preparation of N-(4-(1,3-benzodioxol-5-yl)-3-{2-[(5-chloro-2-pyrimidinyl)oxy]ethoxy}-1-methyl-1H-pyrazol-5-yl)-5-isopropyl-2-pyridinesulfonamide

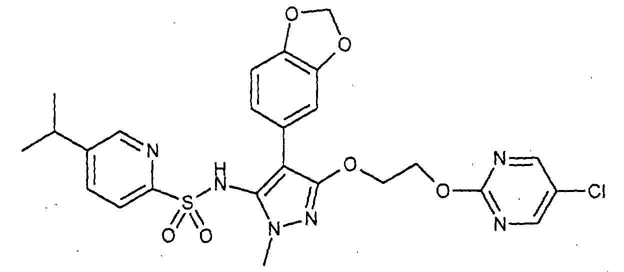

In an oven-dried flask a solution of tetrahydrofuran (50ml) and dimethylacetamide (3ml) was treated with sodium hydride as a 60% dispersion in oil (460mg) under an atmosphere of nitrogen. The reaction was stirred for 5min and then treated with *N*-[4-(1,3-benzodioxol-5-yl)-3-(2-hydroxyethoxy)-1 -methyl-1 *H*-pyrazol-5-yl]-5-isopropyl-2-pyridinesulfonamide (1.0g) (Preparation 13). The reaction was stirred for 3h and was then treated with 5-chloro-2(methylsulfonyl)pyrimidine (460mg). After stirring for a further 3h the reaction mixture was concentrated and the residue was partitioned between water (100ml) and ethyl acetate (100ml). The aqueous was extracted with ethyl acetate (3 X 75ml) and the combined organics were dried over sodium sulfate. The solvent was removed and the crude residue was purified on a silica (40g) column eluting with 20% ether in dichloromethane rising to 45% ether in dichloromethane to yield the title compound as an off-white foam (560mg).

δ_{H} (400MHz, CDCl₃) 1.25 (m, 6H), 2.90 (m, 1H), 3.80 (s, 3H), 4.55 (m, 2H), 4.70 (m, 2H), 5.85 (s, 2H), 6.50 (s, 3H), 7.45 (m, 1H), 7.55 (m, 1H), 8.20 (m, 1H), 8.40 (s, 2H)
^{*m*}/_{*z*} (TSPI) [MH⁺] = 573.5 C₂₅H₂₅N₆O₆ClS + H requires 573.1

### Preparation 13 ― Preparation of N-[4-(1,3-benzodioxol-5-yl)-3-(2-hydroxyethoxy)-1-methyl-1H-pyrazol-5-yl]-5-isopropyl-2-pyridinesulfonamide

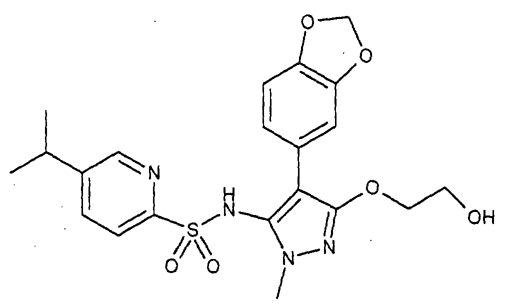

To a solution of 2-[(4-(1,3-benzodioxol-5-yl)-5-(bis[(5-isopropyl-2-pyridinyl)sulfonyl]amino}-1-methyl-1*H*-pyrazol-3-yl)oxy]ethyl acetate (Preparation 14) (9.0g) in ethanol (200ml) was added 2.0M aqueous sodium hydroxide (25ml). The reaction was stirred for 30min and concentrated under reduced pressure and the residue was poured onto 0.5M aqueous citric acid (300ml). This was extracted with ethyl acetate (3 X 80ml) and dried over magnesium sulfate. The solvent was removed under reduced pressure and the crude residue was purified by silica (230g) chromatography eluting with a gradient of 50% ethyl acetate in hexane to 70% ethyl acetate in hexane to afford the title product as a white solid (5.0g)

δ_{H} (400MHz, CDCl₃) 1.25 (6H, s), 2.90 (m, 1H), 3.80 (3H, s), 3.90 (2H, m), 4.30 (2H, m), 5.85 (2H, s), 6.55-6.60 (3H, m), 7.45 (1H, m), 7.60 (1H, m), 8.20 (1H, s)
^{*m*}/_{*z*} (TSPI) [MH⁺] = 461 C₂₁H₂₅N₄O₆S requires 461.1

### Preparation 14 ― Preparation of 2-[(4-(1,3-benzodioxol-5-yl)-5-{bis[(5-isopropyl-2-pyridinyl)sulfonyl]amino}-1-methyl-1H-pyrazol-3-yl)oxy]ethyl acetate

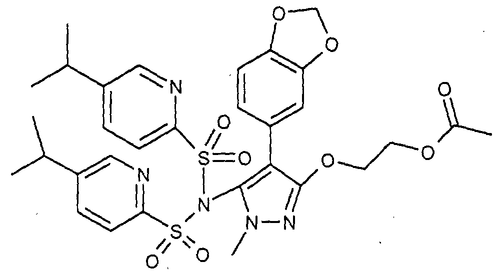

To a solution of 4-dimethylaminopyridine (6.17g) in anhydrous pyridine (100ml) under an atmosphere of nitrogen and at 0°C was added 5-isopropyl-2-pyrindinylsulfonyl chloride (11.1g). The reaction was then treated dropwise at 0°C with a solution of 2-{[5-amino-4-(1,3-benzodioxol-5-yl)-1-methyl-1*H*-pyrazol-3-yl]oxy}ethyl acetate (Preparation 15) (10.8g) in anhydrous pyridine (20ml). The reaction was allowed to warm to room temperature and then stirred for 12h. The reaction mixture was poured into 1.0M aqueous citric acid (500ml) and extracted with ethyl acetate (3 X 200ml). The combined organics were washed with brine (150ml) and dried over magnesium sulfate. The solvent was removed under reduced pressure and the residue was purified on a silica (700g) column using an eluent gradient of 30% ethyl acetate in hexane to 50% ethyl acetate in hexane to afford the title product as a white solid (9.5g).

δ_{H} (400MHz, CDCl₃) 1.30 (12H, s), 2.05 (3H, s), 3.05 (m, 2H), 3.95 (3H, s), 4.35-4.40 (4H, m), 5.85 (2H, s), 6.55 (1H, d), 6.95 (1H, d), 7.05 (1H, s), 7.65 (2H, d), 8.05 (2H, d), 8.40 (2H, s)

### Preparation 15 ― Preparation of 2-{[5-amino-4-(1,3-benzodioxol-5-yl)-1-methyl-1H-pyrazol-3-yl]oxy}ethyl acetate

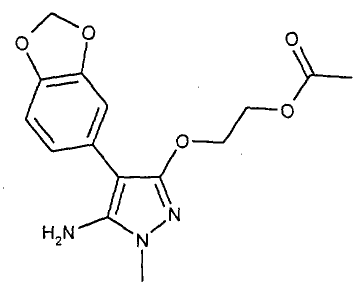

5-amino-4-(1,3-benzodioxol-5-yl)-1-methyl-1*H*-pyrazol-3-ol (Preparation 16) (11.39g) was dissolved in dimethylformamide (40ml), caesium carbonate (15.9g) was added followed by 2-bromoethylacetate (8.16g). The mixture was stirred at room temperature for 16 hours. The mixture was treated with water (750ml) and extracted with ethyl acetate (2x250ml). The organic fractions were combined washed with water (3x350ml), brine (250ml), dried over magnesium sulfate, filtered and evaporated. The crude product was purified on silica (300g) eluting with ethyl acetate : hexane (3:1) to yield the title compound as a white solid (5.57g).

δ_{H} (300MHz, CDCl₃) 6.95 (1H, s), 6.90 (2H, s), 5.95 (2H, s), 4.40 (4H, s), 3.60 (2H, s), 3.50 (3H, s), 2.05 (3H, s).

### Preparation 16 ― Preparation of 5-amino-4-(1,3-benzodioxol-5-vl)-1-methyl-1H-pyrazol-3-ol

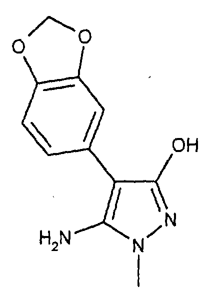

Methylhydrazine (6ml) was added dropwise to a solution of ethyl 2-|(1,3-benzodioxol-5-yl)-2-cyanoacetate (20.0g) in ethanol (100ml) and the mixture was refluxed overnight. The solution was subsequently allowed to cool to room temperature. Concentration to dryness afforded a residue, which was triturated with hot ethanol. The residue was washed with cold ethanol and dried to afford the title compound as a white solid (15.0g).

δ_{H} (400MHz, CDCl₃) 9.45 (1H, br. s), 7.05 (1H, s), 6.90 (1H, d), 6.80 (1H, d), 5.90 (2H, s), 5.65 (2H, br), 3.15 (3H, s).
^{m}/_{z} (TSPI) [MH⁺] = 234.1, C₁₁H₁₂N₃O₃ requires 234.1

### Preparation 17 ― Preparation of 4-(tert-butyl)-N-[3-(2-methoxyethoxy)-1-methyl-4-(4-methylphenyl)-1H-pyrazol-5-yl)benzenesulfonamide

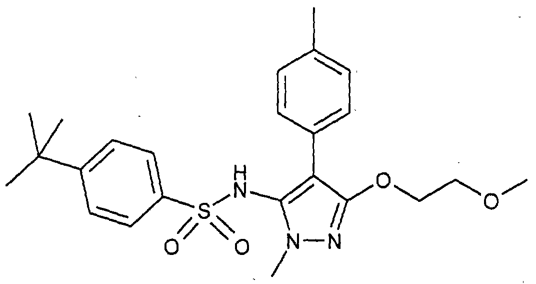

4-(*tert*-Butyl)-*N*-{[4-(*tert*-butyl)phenyl]sulfonyl}-*N*-[3-(2-methoxyethoxy)-1-methyl-4-(4-methylphenyl)-1 H-pyrazol-5-yl]benzenesulfonamide (Preparation 18)(278mg) was dissolved in dioxane, 1M sodium hydroxide solution (4ml) was added and the reaction was stirred at reflux for 0.5 hours. The solvent was removed under reduced pressure. The residue was partitioned between ethyl acetate and 1M hydrochloric acid. The organic phase was separated, dried over magnesium sulphate and the solvent removed under reduced pressure. The product was purified on silica (10g) eluting with a solvent gradient of pentane : ethyl acetate (1:0 to 6.4) to yield the title compound as a white solid (160mg).

δ_{H} (400MHz, CDCl₃) 7.36 (2H, d), 7.12 (2H, d), 6.85 (2H, d), 6.80 (2H, d), 6.55 (1H, s), 4.32 (2H, m), 3.85 (3H, s), 3.66 (2H, m), 3.35 (3H, s), 2.23 (3H, s), 1.24 (9H, s).
^{m}/_{z} (ESI) [MH⁺] =458.0, C₂₄H₃₂N₃O₄S requires 457.1

### Preparation 18 ― Preparation of 4-(tert-butyl)-N-{[4-(tert-butyl)phenyl]sulfonyl}-N-[3-(2-methoxyethoxy)-1-methyl-4-(4-methylphenyl)-1H-pyrazol-5-yl]benzenesulfonamide

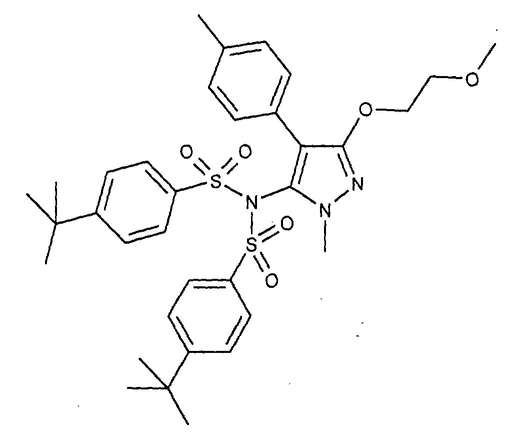

5-Amino-3-(2-methoxyethoxy)-1-methyl-4-(4-methylphenyl)-1H-pyrazole (Preparation 19) (183mg) was dissolved in dichloromethane (5ml) and *tert*-butylbenzenesulfonyl chloride (652mg), potassium hydroxide (550mg) and tetra-n-butylammonium hydrogen sulfate (61mg) were added sequentially. The reaction was sonicated for 3 hours. The solvent was removed under reduced pressure. The residue was dissolved in ethyl acetate (50ml), washed with water (50ml), dried over magnesium sulfate, filtered and evaporated. The residue was purified by column chromatography over silica (20g) eluted with a gradient of pentane : ethyl acetate (1:0 to 1:1) to yield the title compound as a white solid (278mg).
δ_{H} (400MHz, CDCl₃) 7.80 (4H, d), 7.40 (6H, m), 6.90 (2H, d), 4.40 (2H, m), 3.70 (2H, m), 3.40 (3H, s), 3.20 (3H, s), 2.25 (3H, s), 1.35 (18H, s).
^{m}/_{z} (TSPI) [MH⁺] = 654.0, C₃₄H₄₄N₃S₂O₆ requires 654.1

### Preparation 19 ― Preparation of 5-amino-3-(2-methoxyethoxyl)-1-methyl-4-(4-methylphenyl)-1H-pyrazole

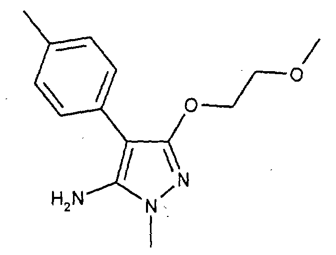

5-amino-1-methyl-4-(4-methylphenyl)-1*H*-pyrazol-3-ol (Preparation 11) (305mg) was dissolved in dimethylformamide (2ml), potassium carbonate (622mg) and 2-bromoethyl methyl ether (208mg) were added. The reaction was stirred at room temperature for 1.5 hours and then at 50°C for 12 hours. The solvent was removed under reduced pressure. The crude product was purified on silica (10g) eluted with a solvent gradient of pentane : ethyl acetate (1:0 to 0:1) to yield the title compound as a white solid (201mg).

δ_{H} (400MHz, CDCl₃) 7.35 (2H, d), 7.20 (2H, d), 4.35 (2H, m), 3.70 (2H, m), 3.65 (2H, s), 3.60 (3H, s), 3.40 (3H, s), 2.35 (3H, s).
^{m}/_{z} (TSPI) [MH⁺] = 262.0, C₁₄H₂₀N₃O₂ requires 262.1

### Preparation 20 ― Preparation of N-(4-(1,3-benzodioxol-5-yl)-3-{2-[(5-bromo-2-pyrimidinyl)oxy]ethoxy}-5-isoxazolyl)-4-(tert-butyl)benzenesulfonamide

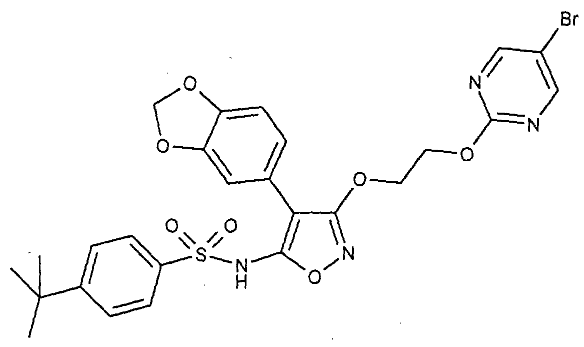

To a solution of *N*-[4-(1,3-benzodioxol-5-yl)-3-(2-hydroxyethoxy)-5-isoxazolyl]-4-(*tert*-butyl)benzenesulfonamide (Preparation 21) (90mg) in tetrahydrofuran (1.5ml), purged with nitrogen three times, was added sodium hydride (16mg of a 60%dispersion in oil) and the reaction mixture stirred for 15 minutes. After which time a solution of 5-bromo-2-chloropyrimidine (41mg) in tetrahydrofuran (0.5ml) was added to the reaction followed by 4-dimethylacetamide (0.1ml). The reaction mixture was left stirring at room temperature overnight. This solution was added to a stirring mixture of ether (30ml) and citric acid (1.0M, 30ml) The organics were separated and further washed with brine (30ml) and dried over magnesium sulfate before being concentrated in vacuo to yield the crude material (90mg). This was purified by HPLC on a 5µ ODS Phenomenex Magellen™ column with a isocratic elution of 0.1M NH40Ac (55%) and acetonitrile (45%) to yield the desired product as a white solid (10mg).

δ_{H} (300MHz, CDCl₃) 8.50(2H, s), 7.85(2H, d), 7.50(2H, d), 6.85(2H, d), 6.75(1H, d), 5.95(2H, s), 4.75(2H, m), 4.65(2H, m), 1.35(9H, s)
^{*m*}/_{*z*} (TSPI) [MH⁺] = 618.5, C₂₆H₂₆BrN₄O₇S requires 617.5

### Preparation 21 ― Preparation of N-[4-(1,3-benzodioxol-5-yl)-3-(2-hydroxyethoxy)-5-isoxazolyl]-4-(tert-butyl)benzenesulfonamide

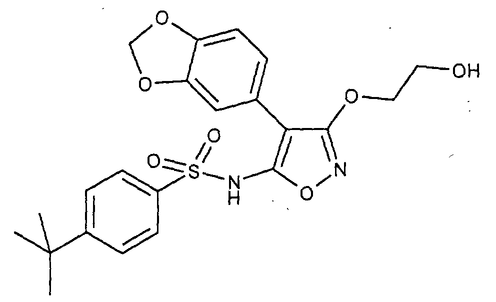

To a stirring solution of 2-({5-[{[4-(*tert*-butyl)phenyl]sulfonyl} (isobutoxycarbonyl)amino]-4-iodo-3-isoxazolyl}oxy)ethyl acetate (Preparation 22) (2.41g) in dioxane (25ml) was added (3,4-methylenedioxyphenyl)boronic acid (0.72g) followed by cesium carbonate (5.15g) and water (3ml). The reaction mixture was purged with nitrogen three times, after which time tetrakis(triphenylphosphine)-palladium(0) (140mg). The reaction mixture was heated to reflux for about 2 hours. Ethanol (50ml) and sodium hydroxide (2M, 50ml) were added to the reaction mixture and then this was stirred at room temperature overnight. The reaction mixture was concentrated in vacuo and the solid residue was partitioned between saturated ammonium chloride solution (100ml) and ethyl acetate (100ml). The organics were washed with brine (50ml), dried over sodium sulfate and concentrated under reduced pressure to yield the crude material as a brown oil. The crude material was purified by column chromatography (100g silica, crude loaded with dichloromethane (10ml)) using a gradient elution of hexane (50% to 0%) and ethyl acetate (50% to 100%) and also with methanol (5% in ethyl acetate) to yield the product as a light brown foam (770mg).

δ_{H} (300MHz, *d*_{*6*}DMSO) 7.65 (2H, d), 7.50 (2H, d), 6.95 (1H, s), 6.90 (2H, d), 6.80 (2H, d), 6.00 (2H, s), 4.10 (2H, t), 3.70 (2H, t), 1.15 (9H, s)

### Preparation 22 ― Preparation of 2-({5-[{[4-(tert-butyl)phenyl]sulfonyl}(isobutoxycarbonyl)amino]-4-iodo-3-isoxazolyl}oxy)ethyl acetate

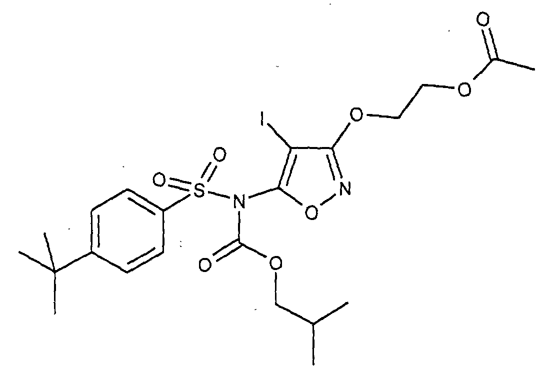

To a stirring solution of 2-{[5-({[4-(*tert*-butyl)phenyl]sulfonyl}amino)-4-iodo-3-isoxazolyl]oxy}ethyl acetate (Preparation 23) (9.44g) in dichloromethane was added pyridine (1.65ml) followed by the slow addition of isobutylchloroforomate (2.41ml) over 10 minutes. The reaction mixture was stirred at room temperature for one hour. The solvent was removed in vacuo to yield the crude material. This was purified using column chromatography (300g silica, compound loaded with dichloromethane (15ml)) using a gradient elution of hexane (100% to 75%) and ethyl acetate (0% to 25%) to yield the desired compound as a yellow oil (7.70g)

δ_{H} (300MHz, CDCl₃) 8.05 (2H, d), 7.60 (2H, d), 4.50 (2H, m), 4.45 (2H, m), 3.90 (2H, d), 2.15 (3H, s), 1.85 (1H, m), 1.35 (9H, s), 0.80 (6H, d)

### Preparation 23 ― Preparation of 2-{[5-({[4-(tert-butyl)phenyl]sulfonyl}amino)-4-iodo-3-isoxazolyl]oxy}ethyl acetate

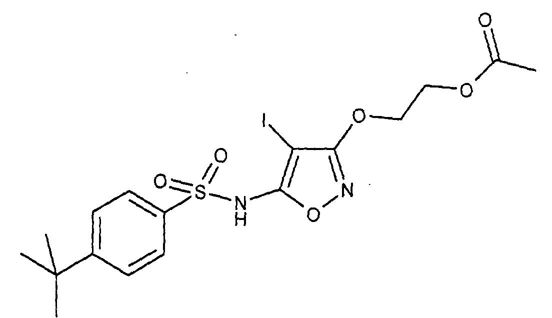

To a stirring solution of 2-{[5-({[4-(*tert*-butyl)phenyl]sulfonyl}amino)-3-isoxazolyl]oxy}ethyl acetate (Preparation 24) (1.17g) in tetrahydrofuran (10ml) was added *N*-iodosuccinimide (0.76g). The reaction mixture was left stirring at room temperature overnight. The solvent was removed *in vacuo* to yield the crude material as a brown oil (1.5g). The crude material was purified using the Biotage™ Flash 40i system (silica, 90g) and eluted with hexane:ethyl acetate (1:9) to yield the product as a brown oil.

δ_{H} (300MHz, CDCl₃) 7.90 (2H, d), 7.55 (2H, d), 4.35 - 4.45 (4H, m), 2.10 (3H, s), 1.35 (9H, s)

### Preparation 24 ― Preparation of 2-{[5-({[4-(tert-butyl)phenyl]sulfonyl}amino)-3-isoxazolyl]oxy}ethyl acetate

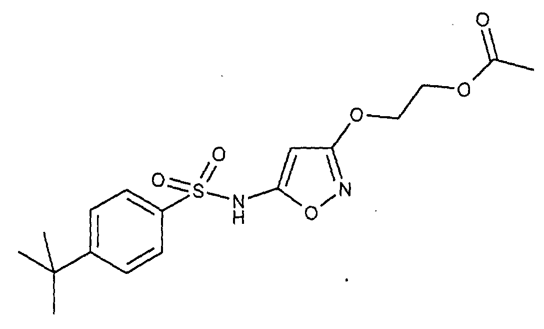

To a solution of *tert*-butyl 3-[2-(acetoxy)ethoxy]-5-({[4-(tert-butyl)phenyl]sulfonyl}amino)-4-isoxazolecarboxylate (Preparation 25) (3.16g) in dichloromethane (40ml) was added trifluoroacetic acid (20ml). The reaction mixture was heated to reflux for 3 hours. After which time the reaction mixture was basified to pH8 using sodium hydrogen carbonate solution and then re-acidified to pH2 using aqueous hydrochloric acid (1.0M). This aqueous layer was extracted into ethyl acetate (2 x 250ml) and the organics combined, dried over magnesium sulfate and the solvent removed *in vacuo* to yield the crude material (2.6g). This was dissolved up in toluene and refluxed for 2 hours. The solvent was removed *in vacuo* to yield the crude material as a whitish brown solid (2.3g). The crude material was purified using the Biotage™ Flash 40i system (silica, 90g) and eluted with hexane:ethyl acetate (5:2) to yield the product as a white solid (1.2g)

δ_{H} (300MHz, CDCl₃) 7.80 (2H, d), 7.55 (2H, d), 5.65 (1H, s), 4.35 - 4.40 (4H, m), 2.10 (3H, s), 1.35 (9H, s)

### Preparation 25 ― Preparation of tert-butyl 3-[2-(acetoxy)ethoxy]-5-({[4-(tert-butyl)phenyl]sulfonyl}amino)-4-isoxazolecarboxylate

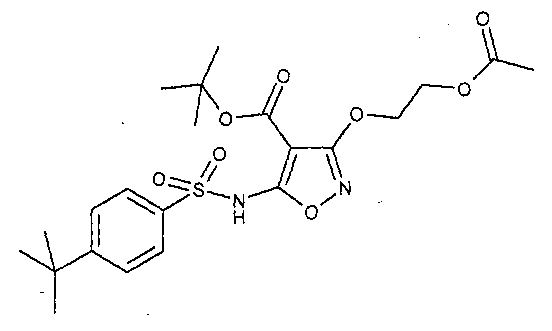

To a stirring solution of *tert*-butyl 3-[2-(acetoxy)ethoxy]-5-amino-4-isoxazolecarboxylate (Preparation 26) (6.0g) in tetrahydrofuran (55ml) under an atmosphere of nitrogen, was added sodium hydride (1.68g of a 60% dispersion in oil). The reaction was stirred for 15 minutes after which time *tert*-butylbenzenesulfonyl chloride (5.14g) was added. The reaction was stirred at room temperature overnight. The solvent was removed *in-vacuo* and redissolved in dichloromethane (50ml) and then washed with water (50ml with 3 drops of HCI). The organics were further washed with brine (40ml), dried over magnesium sulfate and concentrated under reduced pressure to yield the crude material (7.0g). The crude material was purified using the Biotage™ Flash 40i system (silica, 90g) with a gradient elution of ethyl acetate (10% to 95%) and dichloromethane (90% to 5%) to yield the desired product as a white solid (3.5g).

δ_{H} (300MHz, *d*_{*6*}DMSO) 7.75 (2H, d), 7.45 (2H, d), 4.15 - 4.30 (4H, m), 2.00 (3H, s), 1.40 (9H, s), 1.30 (9H, s)

### Preparation 26 ― Preparation of tert-butyl 3-[2-(acetoxy)ethoxy]-5-amino-4-isoxazolecarboxylate

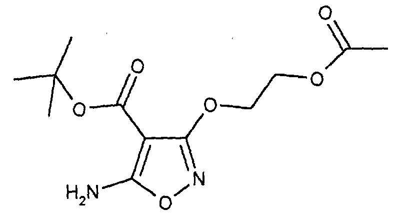

To a stirring solution of *tert*-butyl 5-amino-3-(2-hydroxyethoxy)-4-isoxazolecarboxylate (R.Neidlein, *J.Heterocyclic* Chem.,1989, **26,** 1335) (5.60g) and triethylamine (3.36ml) in tetrahydrofuran (50ml) at room temperature was added dimethylaminopyridine (280mg) followed by acetic anhydride (2.81g). The reaction was stirred for 2 hours at room temperature. The solvent was removed *in vacuo* to yield the crude material (6.0g). The crude material was purified using the Biotage™ Flash 40i System (silica, 90g), eluting with ethyl acetate:hexane (1:1) to yield the product as an off white solid (5.0g)

δ_{H} (300MHz, CDCl₃) 5.80 (2H, br s), 4.40-4.45 (4H, m), 2.10 (3H, s), 1.55 (9H, s);

## Claims

1. A process for making compounds of formula (I)
R-NHSO₂-X-Y-Z (I)
wherein
R is an organic radical;
X is selected from
a) a 5-6 membered monocyclic aromatic ring optionally containing one or two heteroatoms, each independently selected from O, N and S;
b) a C₁-C₆ alkylene group, straight or branched; and
c) a direct link;
Y is -C(CH₃)₂- or -CH(CH₃)-; and
Z is -CH₂OH or -COOH;
which comprises oxidising a compound of formula (II)
R-NHSO₂-X-Y-CH₃ (II)
wherein R, X and Y are as defined above,
with a cytochrome P450 enzyme.

2. A process as claimed in claim 1 wherein the cytochrome P450 enzyme is contained within a microorganism.

3. A process as claimed in claim 2 wherein the microorganism containing the cytochrome P450 enzyme is a unicellular bacteria, a filamentous bacteria or a filamentous fungi.

4. A process as claimed in claims 1-3 wherein R is a a 5-6 membered monocyclic aromatic ring optionally containing one or two heteroatoms, each independently selected from O, N and S, said ring being optionally further substituted.

5. A process as claimed in claim 4 wherein R is selected from one of:
3-[2-(5-bromopyrimidin-2-yl)oxyethoxy]-1-methyl-4-*p*-tolylpyrazol-5-yl,
4-[2-(5-bromopyrimidin-2-yl)oxyethoxy]-5-*p*-tolylpyrimidin-6-yl,
4-(2-hydroxyethoxy)-5-(2-methoxyphenoxy)-2-(2-pyrimidinyl)pyrimidin-6-yl,
4-methoxy-5-(2-methoxyphenoxy)-2-(2-pyrimidinyl)pyrimidin-6-yl,
3-(2-ethoxyethoxy)-1-methyl-4-*p*-tolylpyrazol-5-yl,
4-(1,3-benzod ioxol-5-yl)-3-[2-(5-chloropyrimidin-2-yl)oxyethoxy]-1-methylpyrazol-5-yl,
4-(1,3-benzodioxol-5yl)-3-(2-hydroxyethoxy)-1-methylpyrazol-5-yl,
4-(1,3-benzodioxol-5-yl)-3-[2-(5-bromopyrimidin-2-yl)oxyethoxy]isoxazol-5-yl,
1-methyl-3-(2-methoxyethoxy)-4-p-tolylpyrazol-5-yl,
3-(2-ethoxyethoxy)-1-methyl-4-p-tolylpyrazol-5-yl.

6. A process as claimed in claims 1-5 wherein Z is -CH₂OH.

7. A process as claimed in claim 6 wherein Y is -C(CH₃)₂-.

8. A process as claimed in claim 7 where X is phenylene, pyridylene, ethylene or propylene.

9. A process as claimed in claim 8 where X is 1,4-phenylene.

10. A process as claimed in claim 9 where R is selected from one of:
3-[2-(5-bromopyrimidin-2-yl)oxyethoxy]-1-methyl-4-*p*-tolylpyrazol-5-yl,
4-[2-(5-bromopyrimidin-2-yl)oxyethoxy]-5-*p*-tolylpyrimidin-6-yl,
4-(2-hydroxyethoxy)-5-(2-methoxyphenoxy)-2-(2-pyrimidinyl)pyrimidin-6-yl,
4-methoxy-5-(2-methoxyphenoxy)-2-(2-pyrimidinyl)pyrimidin-6-yl,
3-(2-ethoxyethoxy)-1-methyl-4-*p*-tolylpyrazol-5-yl,
4-(1,3-benzodioxol-5-yl)-3-[2-(5-chloropyrimidin-2-yl)oxyethoxy]-1-methylpyrazol-5-yl,
4-(1,3-benzodioxol-5yl)-3-(2-hydroxyethoxy)-1-methylpyrazol-5-yl,
4-(1,3-benzodioxol-5-yl)-3-[2-(5-bromopyrimidin-2-yl)oxyethoxy]isoxazol-5-yl.

11. A process as claimed in claims 6-10 wherein a compound of formula (II) is treated with one of *Streptomyces lavendulae* ATCC14159, *Streptomyces* sp. PTA-1685, *Streptomyces cyaneus* PTA-1686, *Streptomyces lydicus* PTA-1687, *Streptomyces griseus* ATCC55070, *Streptomyces griseolus* ATCC11796, *Amyclatopsis orientalis* ATCC19795, *Streptomyces griseus* subsp. *griseus* ATCC13273, *Streptomyces argentolus* ATCC11009, *Nocardia meditteranei* ATCC21271, *Streptomyces fumanus* ATCC19904, *Amycoloata autotrophica* ATCC35203, *Streptomyces rimosus* subsp. *rimosus* ATCC10970, *Streptomyces griseus* subsp. *griseus* ATCC10137, *Streptomyces* sp. ATCC31273, *Cunninghamella echinulata* var. *elegans* ATCC8688a, *Mortierella isabellina* ATCC42613, *Verticillium lecanii* ATCC60540, *Mucor circinelloides* ATCC7941, *Cunninghamella echinulata* var. *echinulata* ATCC36190, *Syncephalastrum racemosum* ATCC18192, *Beauvaria sulphurescens* ATCC7159, *Absidia pseudocylindrospora* ATCC24169, *Amycolata autotrophica* ATCC13181, *Rhodococcus rhodochrous* ATCC12674, *Rhodococcus rhodochrous* ATCC19067, *Bacillus megaterium* ATCC14581, *Bacillus megaterium* ATCC13368, *Rhodococcus* sp. ATCC19070, *Actinomyces* sp. ATCC53828, *Bacillus subtilis* ATCC55060, *Pseudomonas putida* ATCC17453, *Pseudomonas putida* ATCC49451, *Bacillus sphaericus* ATCC10208, *Rhizopus oryzae* ATCC11145, *Absidia* blakesleeeana ATCC10148a, *Sepedonium chrysospermum* ATCC13378, *Alcaligenes eutrophus* ATCC17697, Streptomyces *galilaeus* ATCC31133, *Actinoplanes missouriensis* ATCC23342, *Actinoplanes missouriensis* ATCC14538, *Streptomyces peucetius* subsp. *caesius* ATCC27952, *Streptomyces lincolnensis* ATCC25466, *Streptomyces bambergiensis* ATCC 13879, *Streptomyces argillaceus* ATCC 12956, *Streptomyces albogriseolus* ATCC31422, *Streptomyces rutgersensis* ATCC3350, *Corynebacterium* hydrocarboxydans ATCC21767, *Streptomyces fradiae* ATCC10745, *Streptomyces hydrogenans* ATCC19631, Unidentified bacterium ATCC13930, *Actinoplanes* sp. ATCC53771, *Thamnidium elegans* ATCC18191, *Aspergillus terreus* ATCC10020, *Curvularia lunata* ATCC13432, *Emericalla unguis* ATCC13431, *Epicoccum humicola* ATCC12722, *Rhodococcus chlorophenolicus* ATCC49826, *Aspergillus ochraceus* ATCC18500, *Pithomyces cynodontis* ATCC26150, *Streptomyces* roseochromogenes ATCC13400, *Streptomyces griseus* subsp. *autotrophica* ATCC53668, *Streptomyces griseus* subsp. *griseus* ATCC23337, *Absidia* repens ATCC14849 and *Aspergillus alliaceus* ATCC10060.

12. A process as claimed in claim 10 where R is 3-[2-(5-bromopyrimidin-2-yl)oxyethoxy]-1-methyl-4-p-tolylpyrazol-5-yl.

13. A process as claimed in claim 12 where the micro-organism is selected from one of *Syncephalastrum racemosum* ATCC18192, *Streptomyces* sp. PTA-1685, *Streptomyces lavendulae* ATCC14159, *Streptomyces cyaneus* PTA-1686, *Streptomyces griseus* ATCC55070, *Amycolatopsis orientalis* ATCC19795, *Streptomyces argentolus* ATCC11009, *Nocardia meditteranei* ATCC21271, *Streptomyces fumanus* ATCC19904, *Streptomyces rimosus* subsp. *rimosus* ATCC10970, *Streptomyces griseus* ATCC10137, *Cunninghamella echinulata* ATCC8688a, *Mortierella isabellina* ATCC42613, *Verticillium lecanii* ATCC60540, *Mucor circinelloides* ATCC7941 and *Cunninghamella echinulata* ATCC10028b.

14. A process as claimed in claim 10 where R is
4-[2-(5-bromopyrimidin-2-yl)oxyethoxy]-5-*p*-tolylpyrimidin-6-yl.

15. A process as claimed in claim 14 where the micro-organism is selected from one of *Streptomyces lavendulae* ATCC14159, *Streptomyces* sp. PTA-1685, *Streptomyces cyaneus* PTA-1686, *Streptomyces lydicus* PTA-1687, *Streptomyces griseus* ATCC55070, *Streptomyces griseolus* ATCC11796, *Amyclatopsis orientalis* ATCC19795, *Streptomyces griseus* subsp. *griseus* ATCC13273, *Streptomyces fumanus* ATCC19904, *Amycoloata autotrophica* ATCC35203, *Streptomyces griseus* subsp. *griseus* ATCC10137, *Streptomyces* sp. ATCC31273, *Mortierella isabellina* ATCC42613, *Verticillium lecanii* ATCC60540, *Mucor circinelloides* ATCC7941, *Cunninghamella echinulata* var. *echinulata* ATCC36190, *Syncephalastrum racemosum* ATCC18192, *Amycolata autotrophica* ATCC13181, *Rhodococcus rhodochrous* ATCC12674, *Rhodococcus rhodochrous* ATCC19067, *Bacillus megaterium* ATCC14581, *Bacillus megaterium* ATCC13368, *Rhodococcus* sp. ATCC19070, *Actinomyces* sp. ATCC53828, *Bacillus subtilis* ATCC5506, *Pseudomonas putida* ATCC49451, *Bacillus sphaericus* ATCC10208, *Rhizopus oryzae* ATCC11145, *Absidia blakesleeeana* ATCC10148a, *Sepedonium chrysospermum* ATCC13378, *Alcaligenes eutrophus* ATCC17697, *Streptomyces galilaeus* ATCC31133, *Actinoplanes missouriensis* ATCC23342, *Actinoplanes missouriensis* ATCC14538, *Streptomyces peucetius* subsp. *caesius* ATCC27952, *Streptomyces lincolnensis* ATCC25466, *Streptomyces bambergiensis* ATCC13879, *Streptomyces argillaceus* ATCC12956, *Streptomyces albogriseolus* ATCC31422, *Streptomyces rutgersensis* ATCC3350, *Corynebacterium hydrocarboxydans* ATCC21767, *Streptomyces fradiae* ATCC10745, *Streptomyces hydrogenans* ATCC19631, Unidentified bacterium ATCC13930, *Actinoplanes* sp. ATCC53771, *Thamnidium elegans* ATCC18191, *Aspergillus terreus* ATCC10020, *Curvularia lunata* ATCC13432, *Emericalla unguis* ATCC13431 *Epicoccum humicola* ATCC12722, *Rhodococcus chlorophenolicus* ATCC49826, *Aspergillus ochraceus* ATCC18500, *Streptomyces roseochromogenes* ATCC13400, *Streptomyces griseus* subsp. *autotrophica* ATCC53668, *Streptomyces griseus* subsp. *griseus* ATCC23337, *Absidia repens* ATCC14849 and *Aspergillus alliaceus* ATCC 10060.

16. A process as claimed in claim 10 where R is
4-(2-hydroxyethoxy)-5-(2-methoxyphenoxy)-2-(2-pyrimidinyl)pyrimidin-6-yl.

17. The process as claimed in claim 16 where the micro-organism is selected from one of *Streptomyces rimosus* subsp. *rimosus* ATCC10970, *S.fumanus* ATCC 19904, *Streptomyces argentolus* ATCC 11009, *Bacillus megaterium* ATCC14538, *Streptomyces griseus* ATCC13273, *Streptomyces griseus* ATCC10137, *Streptomyces griseolus* ATCC11796, *Streptomyces lavendulae* ATCC14159, *Streptomyces cyaneus* PTA-1686, *Streptomyces* sp. PTA-1685, *Amycolata autotrophica* ATCC13181, *Amycolata autotrophica* ATCC35203 and *Mortierella isabellina* ATCC42613.

18. The process as claimed in claim 10 where R is 4-(1,3-benzodioxol-5-yl)-3-[2-(5-bromopyrimidin-2-yl)oxyethoxy]isoxazol-5-yl.

19. The process as claimed in claim 18 where the micro-organism is selected from one of *Streptomyces rimosus* subsp. *rimosus* ATCC10970, *Streptomyces fumanus* ATCC19904, *Streptomyces argentolus* ATCC11009, *Bacillus megaterium* ATCC14538, *Streptomyces griseus* ATCC13273, *Streptomyces griseus* ATCC10137, *Streptomyces griseolus* ATCC11796, *Streptomyces lavendulae* ATCC14159, *Streptomyces cyaneus* PTA-1686, *Streptomyces* sp. PTA-1685, *Amycolata autotrophica* ATCC13181, *Amycolata autotrophica* ATCC35203 and *Mortierella isabellina* ATCC42613.

20. A process as claimed in claim 6 wherein Y is ―CH(CH₃)-.

21. A process as claimed in claim 20 where X is phenylene, pyridylene, ethylene or propylene.

22. A process as claimed in claim 21 where X is 2,5-pyridylene, wherein Y is at the 2-position.

23. A process as claimed in claim 22 where R is
4-(1,3-benzodioxol-5-yl)-3-[2-(5-chloropyrimidin-2-yl)oxyethoxy]-1-methylpyrazol-5-yl.

24. A process as claimed in claims 20-23 wherein a compound of formula (II) is treated with one of *Streptomyces lavendulae* ATCC14159, *Streptomyces* sp. PTA-1685, *Streptomyces cyaneus* PTA-1686, *Streptomyces lydicus* PTA-1687, *Streptomyces griseus* ATCC55070, *Streptomyces griseolus* ATCC11796, *Amyclatopsis orientalis* ATCC19795, *Streptomyces griseus* subsp. *griseus* ATCC13273, *Streptomyces argentolus* ATCC11009, *Nocardia meditteranei* ATCC21271, *Streptomyces fumanus* ATCC19904, *Amycolata autotrophica* ATCC35203, *Streptomyces rimosus* subsp. *rimosus* ATCC10970, *Streptomyces griseus* subsp. *griseus* ATCC10137, *Streptomyces* sp. ATCC31273, *Cunninghamella echinulata* var. *elegans* ATCC8688a, *Mortierella* *isabellina* ATCC42613, *Verticillium lecanii* ATCC60540, *Mucor circinelloides* ATCC7941, *Cunninghamella echinulata* var. *echinulata* ATCC36190, *Syncephalastrum racemosum* ATCC18192, *Beauvaria sulphurescens* ATCC7159, *Absidia pseudocylindrospora* ATCC24169, *Amycolata autotrophica* ATCC13181, *Rhodococcus rhodochrous* ATCC12674, *Rhodococcus rhodochrous* ATCC19067, *Bacillus megaterium* ATCC14581, *Bacillus megaterium* ATCC13368, *Rhodococcus* sp. ATCC19070, *Actinomyces* sp. ATCC53828, *Bacillus subtilis* ATCC55060, *Pseudomonas putida* ATCC17453, *Pseudomonas putida* ATCC49451, *Bacillus sphaericus* ATCC10208, *Rhizopus oryzae* ATCC11145, *Absidia blakesleeeana* ATCC10148a, *Sepedonium chrysospermum* ATCC13378, *Alcaligenes eutrophus* ATCC17697, *Streptomyces galilaeus* ATCC31133, *Actinoplanes missouriensis* ATCC23342, *Actinoplanes missouriensis* ATCC14538, *Streptomyces peucetius* subsp. *caesius* ATCC27952, *Streptomyces lincolnensis* ATCC25466, *Streptomyces bambergiensis* ATCC13879, *Streptomyces argillaceus* ATCC12956, *Streptomyces albogriseolus* ATCC31422, *Streptomyces rutgersensis* ATCC3350, *Corynebacterium hydrocarboxydans* ATCC21767, *Streptomyces fradiae* ATCC10745, *Streptomyces hydrogenans* ATCC19631, Unidentified bacterium ATCC13930, *Actinoplanes* sp. ATCC53771, *Thamnidium elegans* ATCC18191, *Aspergillus terreus* ATCC10020, *Curvularia lunata* ATCC13432, *Emericalla unguis* ATCC13431, *Epicoccum humicola* ATCC12722, *Rhodococcus chlorophenolicus* ATCC49826, *Aspergillus ochraceus* ATCC18500, *Pithomyces cynodontis* ATCC26150, *Streptomyces roseochromogenes* ATCC13400, *Streptomyces griseus* subsp, *autotrophica* ATCC53668, *Streptomyces griseus* subsp. *griseus* ATCC23337, *Absidia repens* ATCC14849 and *Aspergillus alliaceus* ATCC10060.

25. A process as claimed in claims 1-5 wherein Z is -COOH

26. A process as claimed in claim 25 wherein Y is -C(CH₃)₂-.

27. A process as claimed in claim 26 where X is phenylene, pyridylene, ethylene or propylene.

28. A process as claimed in claim 27 where X is 1,4-phenylene.

29. A process as claimed in claim 28 where R is selected from one of: 3-[2-(5-bromopyrimidin-2-yl)oxyethoxy]-1-methyl-4-*p*-tolylpyrazol-5-yl, 4-[2-(5-bromopyrimidin-2-yl)oxyethoxy]-5-*p*-tolyl-pyrimidin-6-yl, 1-methyl-3-(2-methoxyethoxy)-4-*p*-tolylpyrazol-5-yl, 3-(2-ethoxyethoxy)-1-methyl-4-*p*-tolylpyrazol-5-yl.

30. A process as claimed in claims 25-29 wherein a compound of formula (II) is treated with one of *Amycolata autotrophica* ATCC 35203, *Nocardia meditteranei* ATCC21271, *Amycolatopsis orientalis* ATCC1 19795, *Streptomyces griseolus* ATCC11796, *Streptomyces rimosus* subsp. *rimosus* ATCC10970 and *Nocardia meditteranei* ATCC21271.

31. A process as claimed in claim 29 where R is 3-[2-(5-bromopyrimidin-2-yl)oxyethoxy]-1 -methyl-4-*p*-tolylpyrazol-5-yl.

32. A process as claimed in claim 31 where the micro-organism is selected from one of *Amycolata autotrophica* ATCC 35203, *Nocardia meditteranei* ATCC21271 and *Amycolatopsis orientalis* ATCC19795.

33. A process as claimed in claim in claim 29 where R is 4-[2-(5-bromopyrimidin-2-yl)oxyethoxy]-5-*p*-tolyl-pyrimidin-6-yl.

34. A process as claimed in claim 33 where the micro-organism is selected from one of *Streptomyces griseolus* ATCC11796, *Streptomyces rimosus* subsp. *rimosus* ATCC10970 and *Nocardia meditteranei* ATCC21271.

35. A process as claimed in claim 29 where R is 4-(1,3-benzodioxol-5-yl)-3-[2-(5-bromopyrimidin-2-yl)oxyethoxy]isoxazol-5-yl.

36. A process as claimed in claim 35 where the micro-organism is selected from one of *Streptomyces rimosus* subsp. *rimosus* ATCC10970, *Streptomyces fumanus* ATCC19904, *Streptomyces argentolus* ATCC11009, *Bacillus megaterium* ATCC14538, *Streptomyces griseus* ATCC13273, *Streptomyces* *griseus* ATCC10137, *Streptomyces griseolus* ATCC11796, *Streptomyces lavendulae* ATCC14159, *Streptomyces cyaneus* PTA-1686, *Streptomyces* sp. PTA-1685, *Amycolata autotrophica* ATCC13181, *Amycolata autotrophica* ATCC35203 and *Mortierella isabellina* ATCC42613.

37. A process as claimed in claim 25 wherein Y is -CH(CH₃)-.

38. A process as claimed in claim 37 where X is phenylene, pyridylene, ethylene or propylene.

39. A process as claimed in claim 38 where X is 2,5-pyridylene, wherein Y is at the 2-position.

40. A process as claimed in claims 37-39 wherein a compound of formula II is treated with one of *Amycolata autotrophica* ATCC 35203, *Nocardia meditteranei* ATCC21271, *Amycolatopsis orientalis* ATCC19795 *Streptomyces griseolus* ATCC11796, *Streptomyces rimosus* subsp. *rimosus* ATCC10970 and *Nocardia meditteranei* ATCC21271.
